# EUROPEAN PATENT APPLICATION

(11) **EP 2 366 701 A1**
(43) Date of publication of application: **21.09.2011**
(21) Application number: 09825750.4
(22) Date of filing: 25.10.2009
(51) Int. Cl.: C07D 405/12, C07D 409/12, C07D 209/70, A61K 31/403, A61K 31/54, A61P 43/00, C08B 37/16

(54) **ACENAPHTHO HETEROCYCLE COMPOUNDS, CYCLODEXTRIN INCLUSION COMPOUNDS AND COMPLEXES, AND USES IN THE MANUFACTURES OF BH3 PROTEIN ANALOGUE, BCL-2 FAMILY PROTEIN INHIBITORS THEREOF**

(30) Priority: 11.11.2008 CN 200810228746
(71) Applicant: Dalian University Of Technology, Liaoning 116024 (CN)
(72) Inventor: ZHANG, Zhichao, Dalian Liaoning 116024 (CN); WU, Guiye, Dalian Liaoning 116024 (CN); SONG, Ting, Dalian Liaoning 116024 (CN); XIE, Feibo, Dalian Liaoning 116024 (CN)
(74) Representative: Rippel, Hans Christoph
(86) International application number: PCT/CN2009/074602
(87) International publication number: WO 2010/054575

(57) **Abstract**

The present invention relates to acenaphtho heterocyclic compounds, cyclodextrin inclusion compounds and complexes thereof, and their uses in manufacturing the inhibitors of BH3 analogue, Bcl-2 family proteins. The acenaphtho heterocyclic compounds are obtained by introducing oxo-, thio-, carbonyl, ester or acyl in the 3-, 4- and 6-position of 8-oxo-8*H*-acenaphtho[1,2-b]pyrrole-9-carbonitrile, or further substituting 9-cyano with carboxyl, ester or amide. The compounds can simulate BH3-only protein, competitively binding and antagonizing Bcl-2, Bcl-X_{L} and Mcl-1 proteins in vitro or intracellularly, to induce cell apoptosis. The cyclodextrin inclusion compounds and complexes can improve the effects. Therefore, they can all be used in the manufacture of anticancer compounds.

## Description

### Technical Field of the Invention

The invention relates to a new type of acenaphtho heterocyclic compounds and cyclodextrin inclusion compounds or complexes thereof, prepared using nanotechnology. These compounds can simulate BH3-only protein, competitively binding and antagonizing Bcl-2, Bcl-xL and Mcl-1 proteins in vitro and in vivo, to induce cell apoptosis. Therefore, they can be used as anticancer compounds.

### Background of the Invention

The molecule targeted antitumor drug is becoming a hot spot in new drug research and development and a new generation product for marketization after cytotoxic agents, as an antitumor drug. The Bcl-2 protein is the most important molecular target for antagonizing and reversing the immortality of malignant tumors. Therefore, specifically antagonizing Bcl-2 proteins will achieve the goals of anticancer therapy with high selectivity, safety, high performance and reduced pain by intentionally inducing apoptosis in tumor cells. Among Bcl-2 inhibitors, BH3 analogues (BH3 mimetics) exhibit the most remarkable antitumor effects, the best pharmacodynamic activity and the lowest toxic side effects. In addition, such inhibitors also must have a broad spectrum antagonizing ability on the anti-apoptotic members (including Bcl-2, Bcl-xL and Mcl-1 proteins) of the Bcl-2 family.

However, until now, there are still no marketed antitumor products using Bcl-2 as a target. Among the existing 19 pre-clinical Bcl-2 inhibitors, 3 optimal products are in phase I, phase II and phase III clinical trials respectively, these are ABT-737, researched and developed by Abbott Laboratories, Illinois, USA; Obatoclax (GX15-070) researched and developed by Gemin X; and AT-101, researched and developed by Ascenta in USA. They are all BH3 analogues. The competitive binding constant is up to grade nM with Bcl-2 protein, which is far higher than 15 other similar molecules. However, they all have the following deficiencies: the BH3 analogous level of Gossypol and Obatoclax is insufficient, they are not the absolute BH3 analogue; in other words, they possess cytotoxicity independent of BAX/BAK. This indicates that other target points exist, thus they have toxic side effects. Because of this deficiency, Obatoclax is facing the risk of being eliminated. Although ABT-737 is the absolute BH3 analogue, it cannot react with Mcl-1 and cannot inhibit the Bcl-2 family proteins with broad spectrum, thereby severely limiting its application scope.

The present inventors disclosed a series of acenaphtho heterocyclic compounds of 8-oxo-8*H*-acenaphtho[1,2-b]pyrrole-9-carbonitrile, and disclosed that these compounds had the activity of inhibiting tumor growth through inducing cell apoptosis (Chinese patent, Authorized Announcement No. CN1304370C). However, as a potential antitumor drug on basis of apoptosis, its research and development faces the same difficulties as similar drugs: the complexity of the apoptosis signal gateway, the potential and intense cytotoxicity as well as the inevitable blindness resulting from taking medicine. All of these are the important reasons for the failure in the development of such similar drugs. Therefore, the targeting effect of such drugs should be prominently emphasized in the course of research.

On the other hand, the physicochemical properties of drugs are important influence factors for the onset of the pharmacological effects, and can also influence the accurate evaluation of the pharamacological effects during the development of drugs. Such questions have been noticed during the initial research period. Previously, the study and application of these compounds were severely limited due to lower water solubility.

### Summary of the Invention

The present invention aims to provide acenaphtho heterocyclic compounds, which have stronger targeting and can be used as BH3 analogue, Bcl-2 family protein (including Bcl-2, Bcl-xL and Mcl-1 proteins) inhibitors; and on that basis improve the water solubility and biological availability by combining with contemporary nanotechnology by means of cyclodextrin inclusion or complexation to fully develop their uses as targeting antitumor formations.

The acenaphtho heterocyclic compounds of the present invention have the following structural formula: 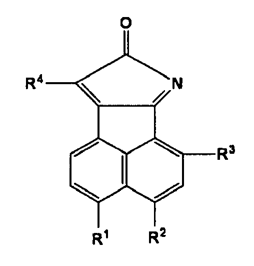 R¹, R², R³ and R⁴ are the substituent groups in the 3-, 4-, 6- and 9-position respectively.
wherein:
(I) R¹=XR⁵, thiophene methoxyl, thiophene methyl amino or thiomorpholinyl, R²=H, R³=H R⁴=CN, COOH, COOR⁶ or CONHR⁷;
(II) R¹=H, R²=XR⁵, thiophene methoxyl, thiophene methylamino or thiomorpholinyl, R³=H, R⁴=CN, COOH, COOR⁶ or CONHR⁷;
(III) R¹=H, R²=H, R³=H, XR⁵, tetrahydropyran-4-oxy-, tetrahydrothiapyran-4-oxy-, thiophene methoxyl, thiophene methylamino or thiomorpholinyl , R⁴=CN;
(IV) R¹=XR⁵ R²=H R³=XR⁵, R⁴=CN;
wherein:
X=O, S, carbonyl, ester or amide;
R⁵= a: (CH₂)ₙAr -(*o,m,p*) Y, Y = CH₃, NO₂ Ph, F, Cl, Br, CF₃, OCH₃, SCH₃ or NH₂; n=0 ∼ 4;
b: tetrahydropyran or tetrahydrothiapyran;
R⁶ = CH₃ or C₂H₅;
R⁷ = CH₃, C₂H₅ or Ar.

The compounds of the present invention can be synthesized by the following two routes:

In the first route, the raw material 8-oxo-8*H*-acenaphtho[1,2-b]pyrrole-9-carbonitrile having excellent rigid, coplanarity and strong electron deficiency undergoes aromatic hydrogenous nucleophilic substitution reaction with the nucleophilic reagents such as alcohol, thioalcohol, phenol or thiophenol, to obtain 3-, 4- or 6-substituted 8-oxo-8*H*-acenaphtho[1,2-b]pyrrole-9-carbonitrile. After the carbonitrile being hydrolyzed, esterified and amidated, the corresponding acid, ester
and amide are obtained. The reaction formula is as follows:

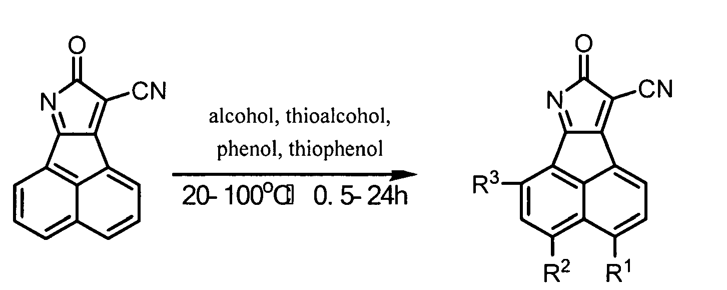

8-oxo-8*H*-acenaphtho[1,2-b]pyrrole-9-carbonitrile in the solvent (tetrahydrofuran, acetonitrile, pyridine, dimethylformamide or dimethyl sulfoxide) reacts with the right amount of nucleophilic reagents such as alcohol, thioalcohol, phenol or thiophenol under the temperature of 20-100°C for 0.5-24 hours. After cooling, some solvent is vaporized out under reduced pressure. Then the product 3 or 6 monosubstituted 8-oxo-8*H*-acenaphtho[1,2-b]pyrrole-9-carbonitrile can be obtained by filtering or direct column chromatography.

In the presence of concentrated sulfuric acid, 3- or 6-monosubstituted 8-oxo-8*H*-acenaphtho[1,2-b]pyrrole-9-carbonitrile can be hydrolyzed to the corresponding acids, then through reacting with the corresponding alcohols and amines, the corresponding esters or amides compounds can be obtained.

In the second route, the raw material acenaphthene quinine and the solvent concentrated sulfuric acid are added into liquid bromine and refluxed for 2 hours to obtain bromoacenaphthene quinine. The resulting bromoacenaphthene reacts with alcohol, thioalcohol, phenol or thiophenol to give the corresponding substituted acenaphthene quinine. The resulting substituted acenaphthene quinine reacts with acetonitrile under weak acid conditions, such as gel silica, to give 3-(2-oxo-2*H*-acenaphthene)-malononitrile. After that, the reaction products are catalyzed by K₂CO₃ and refluxed with acetonitrile for 0.5-6 hours. Then cool and vaporize some solvent under reduced pressure. The corresponding 3- or 4-monosubstituted oxy-8-oxo-8 *H*-acenaphtho[1,2-b]pyrrole-9-carbonitrile is obtained by filtering or direct column chromatography. The following hydrolization, esterification, amidation conditions are the same as that of the first route.

The difference between the first and second route is: the substitution occurs before the looping;, in this way, the two isomers at 3-, 4-position other than at 3-, 6-position can be obtained. The reaction formula is as follows:

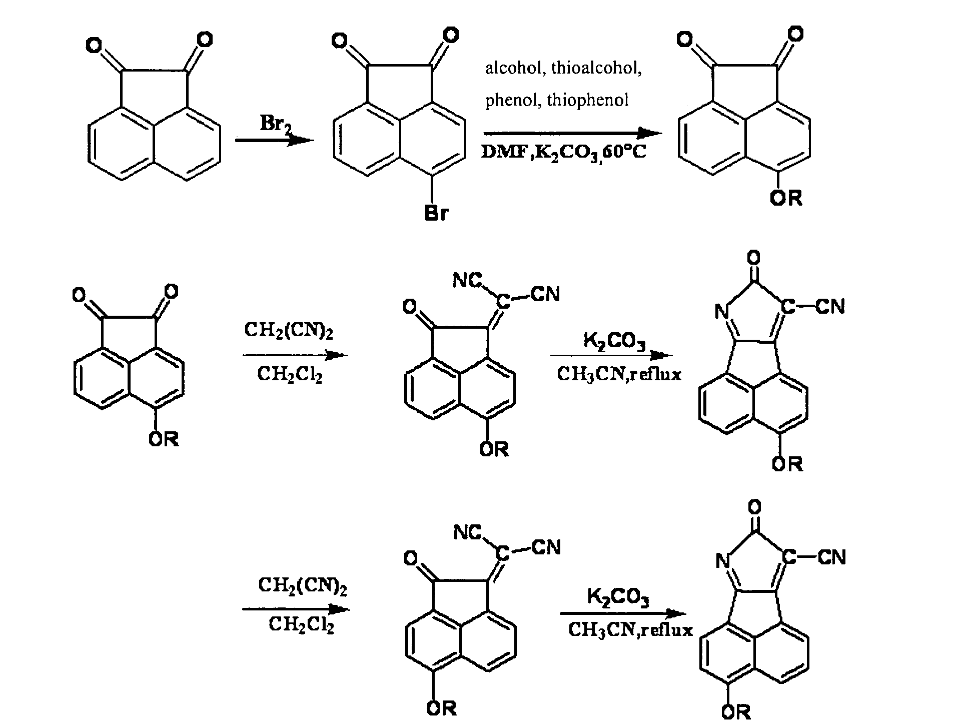

Many methods have been used to detect the BH3 analogous level and the inhibition against Mcl-1 and Bcl-2 for the compounds obtained by the above-mentioned routes. The results demonstrated that the above-mentioned acenaphtho heterocyclic compounds have an extremely high BH3 analogous level, and can effectively inhibit Mcl-1 and Bcl-2 proteins. Because of such attributes, the compounds can be used to prepare BH3 analogue, Bcl-2 family protein inhibitors, and can further be used to prepare antitumor drugs with high targeting.

During the research process of acenaphtho heterocyclic compounds, it has also been found that poor water solubility is the most outstanding issue, which severely limits the study and application of the compounds. Please refer to Modem Technique of Drug Preparation (Cyclodextrin Chemistry-preparation and application, Chemical Industry Press, 2009;β-Cyclodextrin Inclusion Technique and Application, Medicine Innovation Research , 2006, 3(3): 31-33; Chem. Pharm. Bull, 2006, 54(1) 26-32). Another aspect of the present invention is to form inclusion compounds or complexes by including or complexing such compounds with cyclodextrin, thereby improving the water solubility and enhancing the biological availability.

According to the present invention, the cyclodextrin inclusion compounds of the above-mentioned acenaphtho heterocyclic compounds can be prepared by the following method:
① weigh an amount of cyclodextrin and add it into water, then heat under stirring until a saturated solution is formed, wherein the cyclodextrin is β-cyclodextrin, γ-cyclodextrin, 2-hydroxypropyl-β-cyclodextrin, methyl-β-cyclodextrin or hydroxypropyl-γ- cyclodextrin;
② weigh an amount of the acenaphtho heterocyclic compound for inclusion, wherein the mole ratio between the compound and cyclodextrin is 1:3-10;
③ dissolve the acenaphtho heterocyclic compound for inclusion into acetone with a concentration of 5-10 mg/mL, and the resulting solution is added dropwise into the aqueous solution of cyclodextrin in lines, then heat and stir for 1-6 days under the temperature of 40-65°C until a deposition separates out therefrom;
④ filter the above-mentioned solution and wash the filter cake with a small amount of distilled water, then wash out the compounds in free state with a small amount of acetone. After drying under the temperature of 50-70°C and vacuum conditions for 24-48 hours, the cyclodextrin inclusion compound of the acenaphtho heterocyclic compound according to claim 1 is obtained.

According to the present invention, the cyclodextrin complexes of the above-mentioned acenaphtho heterocyclic compounds can be prepared by the following method:
① weigh dry cyclodextrin and the acenaphtho heterocyclic compound to be complexed, the mole ratio between the cyclodextrin and the acenaphtho heterocyclic compounds being 1:1.5-3; wherein the cyclodextrin is β-cyclodextrin, γ-cyclodextrin, 2-hydroxypropyl-β-cyclodextrin, methyl-β-cyclodextrin or hydroxypropyl-γ-cyclodextrin;
② mix the acenaphtho heterocyclic compound to be complexed with N,N'-carbonyldiimidazole with a mole ratio of 1:1-2, and then dissolve them into DMSO until the concentration of the acenaphtho heterocyclic compound to be complexed is 0.2-0.5mmol/mL in DMSO solution, then stir at room temperature for 30-60 minutes;
③ Add the cyclodextrin weighed in step ① and 0.1-0.3 mmol/mL of triethanolamine into the DMSO solution, and then let the reaction last for 18-24 hours at room temperature;
④ add 0.50-1.0 mg/mL of acetone into the reaction system of step 3, the deposition is separated out therefrom under reduced pressure;
⑤ filter, purify and the cyclodextrin complex of the acenaphtho heterocyclic compound of claim 1 is obtained.

The purification can be carried out by ion exchange column. The condition is to adopt Diaion^{™} HP-20 ion exchange resin as adsorbent and a mixed solvent of methanol and water for resolving. The amount of methanol in the mixed solvent is gradually increased and thin-layer chromatography is used to test the elution process. When the amount of methanol in the water in the eluting agent reaches 40-55%, some complexes are obtained by eluting. After the methanol in the resulting elutriant is spin-dried under reduced pressure, the remaining solution is frozen and dried to obtain the complex.

According to the present invention, the resulting cyclodextrin inclusion compounds or complexes of acenaphtho heterocyclic compounds are characterized by the characterization techniques such as phase solubility method, spectrofluorometric method, circular dichroism spectroscopy, infrared spectrometry, thermogravimetric analysis, scanning electron microscope and H nuclear magnetic resonance, mass spectrometry, single crystal X-ray diffraction and so on, and the solubility of the acenaphtho heterocyclic compounds before and after inclusion or complexation and the inhibition against Mcl-1 and Bcl-2 are detected for comparison. The results demonstrate that the treatment methods by using cyclodextrin greatly increase the solubility of the acenaphtho heterocyclic compounds in water, and enhance the inhibitory capability against Bcl-2 and Mcl-1 proteins to some extent. The formulation can also be used to prepare BH3 analogue, Bcl-2 family protein inhibitors, and can further be used to prepare antitumor drugs having high targeting.

Therefore, another objective of the present invention is to provide the use of the above-mentioned acenaphtho heterocyclic compounds, their cyclodextrin inclusion compounds and complexes in manufacturing BH3 analogue, Bcl-2 family protein inhibitors.

The above-mentioned Bcl-2 family protein inhibitors or corresponding antitumor drugs can be the simple substance, cyclodextrin inclusion compounds or complexes of the compounds, or a composition comprising an effective dose of the acenaphtho heterocyclic compounds or cyclodextrin inclusion compounds, complexes thereof and a moderate amount of pharmaceutical adjuvant, and can be made into the desired formulation according to pharmaceutical requirements and the methods for making a pharmaceutical formulation in the prior art.

### Brief Description of Drawings

There are 14 drawings in the present invention, wherein:
Figure 1 is the dynamic curve of the compounds and FAM-Bid peptide competitively binding Bcl-2 protein detected by the fluorescence polarization method;
Figure 2 shows the interactions between Bcl-2 and Bax on a cellular level interfered by the compounds (different concentration);
Figure 3 shows the interactions between Bcl-2 and Bax on a cellular level interfered by the compounds (different action time);
Figure 4 shows the positive results of a BH3 analogous degree of the compounds detected by Bax protein and chondriosome co-localization;
Figure 5 shows the negative results of a BH3 analogous degree of the compounds detected by Bax protein and chondriosome co-localization;
Figure 6 shows the results of the cell toxicity of the compounds depending on BAX/BAK (Gossypol is nonspecific comparison);
Figure 7 is the western blotting electropherogram showing the inhibition of the compounds against Mcl-1;
Figure 8 is the western blotting electropherogram showing the inhibition of the compounds against Bcl-2;
Figure 9 is the semiquantitative curve showing the inhibition of the compound 3-thiomorpholine-8-oxo-8*H*-acenaphtho[1,2-b]pyrrole-9-carbonitrile against Mcl-1 protein;
Figure 10 is the semiquantitative curve showing the inhibition of the compound 3-thiomorpholine-8-oxo-8*H* acenaphtho[1,2-b]pyrrole-9-carbonitrile against Bcl-2 protein;
Figure 11 is the western blotting electropherogram showing the inhibition of acenaphtho heterocyclic compounds and cyclodextrin inclusion compounds and complexes thereof against Mcl-1 and Bcl-2;
Figure 12 is the semiquantitative curve showing the inhibition of the compound 3-thiomorpholine-8-oxo-8*H*-acenaphtho[1,2-b]pyrrole-9-carbonitrile and inclusion compounds thereof against Mcl-1 protein;
Figure 13 is the semiquantitative curve showing the inhibition of the compound 3-thiomorpholine-8-oxo-8*H*-acenaphtho[1,2-b]pyrrole-9-carbonitrile and inclusion compounds thereof against Bcl-2 protein;
Figure 14 is the western blotting electropherogram showing the inhibition of the compounds and inclusion compounds thereof against Mcl-1 in an in vivo tumor model, wherein: 1. blank control group; 2. control groups ① ;3. control group ②; 4. experimental group ① ; 5. experimental group ②; 6. experimental group ③; 7. experimental group ③.

### Detailed Description of Preferred Embodiments

Various embodiments of the present invention will now be described more fully with reference to the accompanying drawings.

### Part I: Preparation and Characterization of Acenaphtho Heterocyclic Compounds

### Example 1: Synthesis and Characterization of 8-oxo-8H-acenaphtho[1,2-b] pyrrole-9-carbonitrile

0.1mol of acenaphthene quinone, 0.11mol of malononitrile and 150mL of acetonitrile were added into a 500mL single neck flask in sequence. The reaction mixture was heated under reflux for 4 hours until the color turned into transparent orange red from cloudy pale yellow. After that, the reaction mixture was cooled to room temperature, filtered, and the orange red filter cake was collected to obtain 1-dicyanomethylene-2-oxo-acenaphthene. 0.05 mol of 1-dicyanomethylene-2-oxo-acenaphthene, 1g of K₂CO₃, and 200mL of acetonitrile were added into a 500mL single neck flask in sequence, the reaction mixture was heated under reflux for 4 hours. A large amount of earth yellow solid separated out. Filtered and the filter case was collected and washed with a large quantity of warm water, and then dried and weighed; the yield was 95%.
M.p. 275-277°C; ¹H NMR (400M, DMSO): δ 8.705(d, J=8.0Hz , 1H), 8.662 (d, J=8.8Hz, 1H), 8.631 (d, J=8.0Hz, 1H), 8.411 (d, J=8.0Hz, 1H), 8.06 (t, J=8.0Hz, 1H), 7.984 (t,J=8.0Hz, 1H).

### Example 2: Synthesis and Characterization of 3-(4-methylphenoxy)-8-oxo-8H-acenaphtho[1,2-b]pyrrole-9-carbonitrile

1g of 8-oxo-8*H*-acenaphtho[1,2-b]pyrrole-9-carbonitrile and 0.47g of p-methylphenol were added into 50mL acetonitrile. The mixture was refluxed and stirred for 3 hours. Some solvent was vaporized out. The product 3-(4-methylphenoxy)-8-oxo-8*H-*acenaphtho[1,2-b]pyrrole-9-carbonitrile was obtained through a chromatographic column with a yield of 40%.

Structure determination results: M.p. 232-233°C; 1H NMR (400M, CDCl3): δ 8.916 (dd, J=8.8Hz 1H), 8.623 (d, J=8.8Hz, 1H), 8.447 (d, J=6.4Hz, 1H), 7.859 (t, J=8.0Hz, 1H), 8.324 (d, J=8.4Hz, 2H), 7.101 (d, J=8.4Hz, 2H), 7.016 (d, J=8.4Hz, 1H) ,3.256 (s, 3H).

### Example 3: Synthesis and Characterization of 3-phenoxy-8-oxo-8H-acenaphtho [1,2-b]pyrrole-9-carbonitrile (A) and 4-phenoxy-8-oxo-8H-acenaphtho[1,2-b] pyrrole-9-carbonitrile (B)

0.93g of phenoxy acenaphthene quinone and 0.3g of malononitrile was weighed and dissolved in dichloromethane. The mixture was applied to a gel silica column and eluted quickly. After all of the mixture passed through, the column was spin-dried. Red solid was obtained with a weight of 10.1 g and a yield of 92%. 0.08g of K₂CO₃ and 20mL of acetonitrile were added into 0.6g of 3-phenoxy-(2-oxo-2*H*-acenaphthene)-malononitrile. The mixture was heated and refluxed for 3 hours. After the reaction was completed, the reaction solution was spin-dried and separated by chromatographic column (CH₂Cl₂: petroleum ether = 2:1) to obtain an orange red solid. The isomer ratio of 1: 0.3 was tested by nuclear magnetic resonance. The resulting isomers were separated by liquid phase separation to obtain two isomers.

The first component A: M.p. 265-267°C; ¹H NMR (400M, CDCl₃): δ 8.927 (d, J=8.0Hz, 1H), 8.630 (d, J=8.8Hz, 1H), 8.450 (d, J=7.2Hz, 1H), 7.876 (t, J=8.0Hz, 1H),7.754 (t, J=8.0Hz,2H), 7.392 (t, J=7.6Hz, 1H), 7.233 (d, J=7.6Hz, 2H), 7.028 (d, J=8.4Hz,1H).

The second component B: M.p. 282-283°C; ¹H NMR (400M, CDCl₃): δ 9.047 (dd, J=8.0Hz, 1H), 8.850 (dd, J=7.6Hz, 1H), 8.213 (d, J=8.2Hz, 1H), 7.999 (t, J=8.0Hz,1H),7.561 (t, J=8.0Hz,2H), 7.410 (t, J=7.0Hz, 1H), 7.251 (d, J=8.8Hz, 2H), 6.899 (d, J=8.4Hz,1H).

### Example 4: Synthesis and Characterization of 3-(p-methylphenoxy)-8-oxo-8H acenaphtho[1,2-b]pyrrole-9-carbonitrile (A) and 4-(p-methylphenoxy)-8-oxo-8H-acenaphtho[1,2-b]pyrrole-9-carbonitrile (B)

1g of *p*-methylphenoxy acenaphthene quinone and 0.3g of malononitrile were dissolved into dichloromethane. The mixture was applied to a gel silica column and eluted quickly. After the whole mixture passed through, the column was spin-dried. A red solid was obtained with a weight of 11.2g and a yield of 93%. 0.08g of K₂CO₃ and 20mL of acetonitrile were added into 0.7g of 3-phenoxy-(2-oxo-2*H*-acenaphthene)- malononitrile. The mixture was heated and refluxed for 4 hours. After the reaction finished, the reaction solution was spin-dried and separated by chromatographic column (CH₂Cl₂: petroleum ether = 1:1) to obtain an orange red solid. The isomer ratio is 1: 0.4 tested by nuclear magnetic resonance. The resulting isomers were separated by liquid phase separation to obtain two isomers.

The first component A: M.p. 232-233°C; ¹H NMR (400M, CDCl₃): δ 8.916 (dd, J=8.8Hz, 1H), 8.623 (d, J=8.8Hz, 1H), 8.447 (d, J=6.4Hz, 1H), 7.859 (t, J=8.0Hz, 1H), 8.324 (d, J=8.4Hz, 2H), 7.101 (d, J=8.4Hz, 2H), 7.016 (d, J=8.4Hz, 1H), 2.351(s, 3H).

The second component B: M.p. 258-260°C; ¹H NMR (400M, CDCl₃): δ 8.987 (dd, J=8.8Hz, 1H), 8.858 (d, J=8.8Hz, 1H), 8.208 (d, J=8.4Hz, 1H), 7.986 (t, J=8.0Hz, 1H), 8.333 (d, J=8.4Hz, 2H), 7.112 (d, J=8.4Hz, 2H), 6.889 (d, J=8.4Hz, 1H),2.349 (s, 3H).

### Example 5: Synthesis and Characterization of 3-(m-methylphenylthio)-8-oxo-8H-acenaphtho[1,2-b]pyrrole-9-carbonitrile (A) and 4-(m-methylphenylthio) -8-oxo-8H-acenaphtho[1,2-b] pyrrole-9-carbonitrile (B)

1g of *m*-methylphenylthio acenaphthene quinone and 0.3g of malononitrile were dissolved into dichloromethane. The mixture was applied to a gel silica column and eluted quickly. After the whole mixture passed through, the column was spin-dried. A red solid was obtained with a weight of 12.2g and a yield of 91%. 0.08g of K₂CO₃ and 20mL of acetonitrile were added into 0.7g of 2-phenylthio-(2-oxo-2*H*-acenaphthene)- malononitrile. The mixture was heated and refluxed for 4 hours. After the reaction was completed, the reaction solution was spin-dried and separated by a chromatographic column (CH₂Cl₂: petroleum ether = 1:1) to obtain a red solid. The isomer ratio was 1: 0.25 as tested by nuclear magnetic resonance. The resulting isomers were separated by liquid phase separation to obtain two isomers.

The first component A: M.p. 255-257°C; ¹H NMR (400M, CDCl₃): δ 8.826 (dd, J=8.8Hz, 1H), 8.513 (d, J=8.8Hz, 1H), 8.327 (d, J=6.4Hz, 1H), 7.659 (t, J=8.0Hz, 1H), 8.014 (d, J=8.4Hz, 2H), 6.901 (d, J=8.4Hz, 2H), 6.896 (d, J=8.4Hz, 1H),2.353(s, 3H).

The second component B: M.p. 269-271°C; ¹H NMR (400M, CDCl₃): δ 8.877 (dd, J=8.8Hz, 1H), 8.748 (d, J=8.8Hz, 1H), 8.108 (d, J=8.4Hz, 1H), 7.856 (t, J=8.0Hz, 1H), 8.123 (d, J=8.4Hz, 2H), 6.892 (d, J=8.4Hz, 2H), 6.679 (d, J=8.4Hz, 1H),2.355(s, 3H).

### Example 6: Synthesis and Characterization of 6-(thienyl-2-methoxy)-8-oxo-8H-acenaphtho[1,2-b]pyrrole-9-carbonitrile

1g of 8-oxo-8*H*-acenaphtho[1,2-b]pyrrole-9-carbonitrile and 0.5g of thienylmethanol were added into 50mL acetonitrile. The mixture was refluxed and stirred for 3 hours. solvent was vaporized out. The product 6-(2-thienylmethoxy)-8-oxo-8H-acenaphtho[1,2-b]pyrrole-9-carbonitrile was obtained through a chromatographic column with a yield of 45%.
M.p. 241-243°C; ¹H NMR (400M, CDCl₃): δ 8.685 (dd, J=8.7Hz , 1H), 8.433 (d, J=8.7Hz, 1H), 8.014 (d, J=6.4Hz, 1H), 7.75 (t, J=8.0Hz, 1H), 7.251 (t, J=8.4Hz, 1H), 7.181 (d, J=8.4Hz, 1H), 6.985 (d, J=8.4Hz, 1H), 6.232 (d, J=8.4Hz, 1H) ,3.454(s, 2H).

### Example 7: Synthesis and Characterization of 3-(3-fluorophenylcarbonyl) -8-oxo-8H-acenaphtho[1,2-b]pyrrole-9-carbonitrile (A) and 4-(3-fluoro phenylcarbonyl)-8-oxo-8H-acenaphtho[1,2-b]pyrrole-9-carbonitrile (B)

1g of ***m***-fluorophenylcarbonyl acenaphthene quinone and 0.4g of malononitrile were dissolved into dichloromethane. The mixture was applied to a gel silica column and eluted quickly. After the whole mixture passed through, the column was spin-dried. A cardinal red solid was obtained with a weight of 10.5g and a yield of 85%. 0.08g of K₂CO₃ and 20mL of acetonitrile were added into 0.8g of 2-fluorocarbonyl-(2-oxo-2*H*-acenaphthene)-malononitrile and the mixture was heated and refluxed for 3 hours. After the reaction finished, the reaction solution was spin-dried and separated by a chromatographic column (CH₂Cl₂: petroleum ether = 2:1) to obtain a cardinal red solid. The isomer ratio is 1: 0.2 tested by nuclear magnetic resonance.

The resulting isomers were separated by liquid phase separation to obtain two isomers.

The first component A: M.p. 285-287°C; ¹H NMR (400M, CDCl₃): δ 8.726 (dd, J=8.8Hz, 1H), 8.423 (d, J=8.8Hz, 1H), 8.015 (d, J=6.4Hz, 1H), 7.598 (t, J=8.0Hz, 1H), 8.003 (d, J=8.4Hz, 2H), 6.853 (d, J=8.4Hz, 2H), 6.756 (d, J=8.4Hz, 1H).

The second component B: M.p. 269-271°C; ¹H NMR (400M, CDCl₃): δ 8.568 (dd, J=8.8Hz, 1H), 8.478 (d, J=8.8Hz, 1H), 8.006 (d, J=8.4Hz, 1H), 7.568 (t, J=8.0Hz, 1H), 8.045 (d, J=8.4Hz, 2H), 6.908(d, J=8.4Hz, 2H), 6.596 (d, J=8.4Hz, 1H).

### Example 8: Synthesis and Characterization of 3-(N-phenylamido)-8-oxo-8H-acenaphtho[1,2-b]pyrrole-9-carbonitrile (A) and 4-(N-phenylamido)-8-oxo-8H-acenaphtho[1,2-b]pyrrole-9-carbonitrile (B)

1g of phenylamide acenaphthene quinone and 0.4g of malononitrile were dissolved into dichloromethane. The mixture was applied to a gel silica column and eluted quickly. After the whole mixture passed through, the column was spin-dried. A cardinal red solid was obtained with a weight of 11.2g and a yield of 86%. 0.08g of K₂CO₃ and 20mL of acetonitrile were added into 0.8g of phenylamide-(2-oxo-2*H-*acenaphthene)-malononitrile. The mixture was heated and refluxed for 3 hours. After the reaction finished, the reaction solution was spin-dried and separated by a chromatographic column (CH₂Cl₂: petroleum ether = 2:1) to obtain a cardinal red solid. The isomer ratio is 1: 0.4 as tested by nuclear magnetic resonance. The resulting isomers were separated by liquid phase separation to obtain two isomers.

The first component A: M.p. 281-283°C; ¹H NMR (400M, CDCl₃): δ 9.112 (d, J=8.0Hz, 1H), 8.945 (d, J=8.8Hz, 1H), 8.682 (d, J=7.2Hz, 1H), 8.452 (t, J=8.0Hz, 1H), 8.312 (s, 1H),7.986 (t, J=8.0Hz,2H), 7.627 (t, J=7.6Hz, 1H), 7.433 (d, J=7.6Hz, 2H), 7.241 (d, J=8.4Hz,1H).

The second component B: M.p. 293-294°C; ¹H NMR (400M, CDCl₃): δ 9.213 (dd, J=8.0Hz, 1H), 9.012 (dd, J=7.6Hz, 1H), 8.685 (d, J=8.2Hz, 1H), 8.428 (t, J=8.0Hz, 1H), 8.320 (s, 1H),7.896 (t, J=8.0Hz,2H), 7.675 (t, J=7.0Hz, 1H), 7.531 (d, J=8.8Hz, 2H), 7.015 (d, J=8.4Hz, 1H).

### Example 9: Synthesis and Characterization of 3-(tetrahydro-2H-pyranyl-4-oxy) -8-oxo-8H-acenaphtho[1,2-b]pyrrole-9-carbonitrile (A) and 4-(tetrahydro-2H-pyranyl-4-oxy)-8-oxo-8H-acenaphtho[1,2-b] pyrrole-9-carbonitrile (B)

1.0g of tetrahydropyranyloxyl acenaphthene quinone and 0.4g of malononitrile were dissolved into dichloromethane. The mixture was applied to a gel silica column and eluted quickly. After the whole mixture passed through, the column was spin-dried. A cardinal red solid was obtained with a weight of 11.2g and a yield of 86%. 0.08g of K₂CO₃ and 20mL of acetonitrile were added into 0.8g of 4-tetrahydropyranyl-(2-oxo-2*H*-acenaphthene)-malononitrile. The mixture was heated and refluxed for 9 hours. After the reaction was completed, the reaction solution was spin-dried and separated by a chromatographic column (CH₂Cl₂: petroleum ether = 2:1) to obtain a deep red solid. The isomer ratio of 1: 0.4 tested by nuclear magnetic resonance. The resulting isomers were separated by liquid phase separation to obtain two isomers.

The first component A: M.p. 230-231°C; ¹H NMR (400M, CDCl₃): δ 8.601 (d, J=8.0Hz, 1H), 8.134 (d, J=8.8Hz, 1H), 7.945 (dd, J=8.0Hz, 1H), 7.452 (d, J=8.4Hz,1H), 3.822 (t, J=4.8Hz ,4H),3.815(t, J=5.0Hz ,4H),3.766 (t, J=5.2Hz,1H).

The second component B: M.p. 242-244°C; ¹H NMR (400M, CDCl₃): δ 8.568 (d, J=8.0Hz, 1H), 8.115 (d, J=8.8Hz, 1H), 7.856 (dd, J=8.0Hz, 1H), 7.326 (d, J=8.4Hz,1H), 3.796 (t, J=4.8Hz ,4H),3.807(t, J=5.0Hz ,4H),3.791 (t, J=5.2Hz, 1H).

### Example 10: Synthesis and Characterization of 3-phenoxy-8-oxo-8H-acenaphtho [1,2-b]pyrrole-9-carboxylic acid

60ml of concentrated sulfuric acid or 25ml of fuming sulfuric acid were added into a 50ml single neck flask. 0.05mol of 3-phenoxy-8-oxo-8*H*-acenaphtho[1,2-b]pyrrole-9-carbonitrile was added thereinto in batches at a temperature of 0-5°C within 1 hour. After that, the reaction was carried out for another 16 hours at room temperature, and the resulting reaction mixture was viscous, deep, brownish red. Then the resulting mixture was dropped slowly into crushed ice and stirred acutely. After that, the mixture was stood and filtered. The filter cake was washed with a large quantity of water until it became neutral. The filter cake was dried to obtain the deep yellow product with a yield of 96%.
M.p. 248°C; ¹H NMR (400M, CDCl₃): δ 11.42 (s,1H),8.965 (dd, J=8.0Hz, 1H), 8.750 (dd, J=7.8Hz, 1H), 8.313 (d, J=8.2Hz, 1H), 7.999 (t, J=8.2Hz,1H),7.561 (t, J=8.2Hz,2H), 7.410 (t, J=7.0Hz, 1H), 7.251 (d, J=8.8Hz, 2H), 6.963 (d, J=8.4Hz, 1H).

### Example 11: Synthesis and Characterization of 3-phenoxy-8-oxo-8H-acenaphtho [1,2-b]pyrrole-9-carboxylate

0.01mol of 3-phenoxy-8-oxo-8*H*-acenaphtho[1,2-b]pyrrole-9-carbonitrile, 50ml of acetonitrile as solvent, 0.02mmol of K₂CO₃ as neutralizing reagent and iodomethane over ten times were added into a 100ml single neck flask in sequence. Under nitrogen protection, the mixture was heated up to 40°C and the reaction was lasted for 15 hours. The acetonitrile was vaporized out under reduced pressure, and the reactant was fully dissolved by addition of dichloromethane. After filtration, the filtrate was spin-dried to obtain a yellow brown crude product. The deep yellow product was obtained by column chromatographic separation with gel silica (developing agent: dichloromethane-methanol=40: 1). The yield was 92%.
M.p. 213°C; ¹H NMR (400M, CDCl₃): δ 9.102 (d, J=7.2Hz,1H), 8.965 (dd, J=8.0Hz, 1H), 8.850 (dd, J=7.8Hz, 1H), 8.233 (d, J=8.2Hz, 1H), 7.856(t, J=8.2Hz,1H),7.453 (t, J=8.2Hz,2H), 7.350 (t, J=7.2Hz, 1H), 7.325 (d, J=8.4Hz, 2H), 3.213 (s, 3H).

### Example 12: Synthesis and Characterization of 3-phenoxy-8-oxo-8H-acenaphtho [1,2-b] pyrrole-9-N-tert-butylamide

0.01 mol of 3-phenoxy-8-oxo-8*H*-acenaphtho[1,2-b]pyrrole-9-carboxylic acid, 50ml of DMF as solvent, 0.02mmol of triethylamine, 0.01mmol of (EtO)₂P(=O)CN and tert-butylamide over ten times were added into a 100ml single neck flask in sequence and reacted for 1 hour at room temperature. Then a yellow solid was obtained after the reaction finished. The yield was 85%. M.p. 237°C; ¹H NMR (400M, CDCl₃): δ8.746 (dd, J=8.0Hz, 1H), 8.650 (dd, J=7.8Hz, 1H), 8.213 (d, J=8.2Hz, 1H), 7.846 (t, J=8.0Hz,1H),7.352 (t, J=8.0Hz,2H), 7.210 (t, J=7.4Hz, 1H), 7.051 (d, J=8.4Hz, 2H), 6.963 (d, J=8.4Hz,1H), 3.721(s, 3H) ,3.113 (m, 2H), 1.568(dd, J=5.6Hz ,2H), 1.421(m, J=5.7Hz ,2H), 0.968(t, J=6.2Hz ,3H).

### Example 13: Synthesis and Characterization of 3-(4-bromophenylthio)-8-oxo-8H-acenaphtho[1,2-b]pyrrole-9-carbonitrile

1g of 8-oxo-8*H*-acenaphtho[1,2-b]pyrrole-9-carbonitrile and 3.2g of 4-bromothiophenol were added into 50mL acetonitrile and reacted for 2 hours at room temperature. Some solvent was vaporized out. The compound 3-(4-bromophenylthio)-8-oxo-8*H*-acenaphtho[1,2-b]pyrrole-9-carbonitrile was obtained through a chromatographic column with a yield of 40%.
M.p. 262-263°C; 1H NMR (400M, CDCl3): δ 8.852 (dd, J=8.8Hz, 1H), 8.813 (d, J=8.8Hz, 1H), 8.015 (d, J=6.4Hz, 1H), 7.945 (t, J=8.0Hz, 1H), 7.560 (d, J=8.4Hz, 2H), 7.096 (d, J=8.4Hz, 2H), 7.006 (d, J=8.4Hz, 1H).

### Example 14: Synthesis and Characterization of 3,6-di(4-bromophenylthio)-8-oxo-8H-acena phtho[1,2-b]pyrrole-9-carbonitrile

1g of 8-oxo-8*H*-acenaphtho[1,2-b]pyrrole-9-carbonitrile and 6.5g of 4-bromothiophenol were added into 50mL acetonitrile and reacted for 36 hours at room temperature. Some solvent was vaporized out. The compound 3,6-di(4-bromophenylthio)-8-oxo-8*H*-acenaphtho[1,2-b]pyrrole-9-carbonitrile was obtained through a chromatographic column with a yield of 20%.
M.p. 262-263 °C ; 1H NMR (400M, CDCl3): δ 8.815 (dd, J=8.8Hz , 1H), 8.671 (d, J=8.8Hz, 1H), 7.881 (t, J=6.4Hz, 1H), 7.551(q, J=8.0Hz, 4H), 7.215 (q, J=8.4Hz, 4H), 6.472 (s, 1H).

### Example 15: Synthesis and Characterization of 6-(4-aminophenylthio)-8-oxo-8H-acenaphtho[1,2-b]pyrrole-9-carbonitrile

1g of 8-oxo-8*H*-acenaphtho[1,2-b]pyrrole-9-carbonitrile and 2.5g of 4-aminothiophenol were added into 50mL acetonitrile and reacted for 2 hours at room temperature. solvent was vaporized out. The compound 6-(4-aminophenylthio)-8-oxo-8H-acenaphtho[1,2-b]pyrrole-9-carbonitrile was obtained through a chromatographic column with a yield of 30%. M.p. 255-257°C; 1H NMR (400M, CDCl3): δ 8.832 (dd, J=8.8Hz, 1H), 8.801 (d, J=8.8Hz, 1H), 7.985 (d, J=6.4Hz, 1H), 7.925 (t, J=8.0Hz, 1H), 7.570 (d, J=8.4Hz, 2H), 6.997 (d, J=8.4Hz, 2H), 7.006 (d, J=8.4Hz, 1H), 6.271 (s, 2H).

### Example 16: Synthesis and Characterization of 3,6-di(4-aminophenylthio)-8-oxo-8H-acenaphtho[1,2-b]pyrrole-9-carbonitrile

1g of 8-oxo-8*H*-acenaphtho[1,2-b]pyrrole-9-carbonitrile and 5.0g of 4-aminothiophenol were added into 50mL acetonitrile and reacted for 30 hours at room temperature. Some solvent was vaporized out. The compound 3,6-di(4-aminophenylthio)-8-oxo-8*H*-acenaphtho[1,2-b]pyrrole-9-carbonitrile was obtained through a chromatographic column with a yield of 25%. M.p. 288-290°C; 1H NMR (400M, CDCl3): δ 8.835 (dd, J=8.8Hz, 1H), 8.682 (d, J=8.8Hz, 1H), 7.973 (t, J=6.4Hz, 1H), 7.581(q, J=8.0Hz, 4H), 7.234 (q, J=8.4Hz, 4H), 6.651 (s, 1H) , 6.269 (s, 4H).

### Part II: Preparation and Characterization of Cyclodextrin Inclusion Compounds and Complexes of Acenaphtho Heterocyclic Compounds

This part involves the preparation and characterization of cyclodextrin inclusion compounds and complexes of acenaphtho heterocyclic compounds. The used characterization methods comprise ultraviolet spectroscopy, spectrofluorometric method, circular dichroism spectroscopy, infrared spectroscopy, thermogravimetric analysis and SEM. Please refer to the following resources for the instruments and detection methods in this part if no specific explanation is given:

Phase solubility diagram: drawn according to the method described in J. Agric. Food Chem., 2007, 55 (9), 3535-3539.

Ultraviolet spectroscopy: HP8453 (America); please refer to Journal of Photochemistry and Photobiology A: Chemistry 173 (2005) 319-327 for the detection method.

Spectrofluorometric method: PTI-700 (America); please refer to J Fluoresc (2008) 18:1103-1114 for the detection method.

Circular dichroism spectroscopy: J-810 (Japan); please refer to J. Phys. Chem. B, 2006, 110 (13), 7044-7048 for the detection method.

Infrared spectroscopy: FT/IR-430 (Japan); please refer to Mol. Pharmaceutics, 2008, 5 (2), 358-363 for the detection method.

Thermogravimetric analysis: TGA/SDT851e (Switzerland); please refer to Mol. Pharmaceutics, 2008, 5 (2), 358-363 for the detection method.

SEM: JSM-5600LV (Japan); please refer to J. Med. Chem. 2003, 46, 4634-4637 for the detection method.

H NMR: Bruker AvanceII 400M (Switzerland); the detection condition is (solvent CDCl3, 400M).

Mass spectroscopy: GC-Tof MS (Britain); please refer to J. Org. Chem. 2000, 65, 9013-9021 for the detection method.

Single-crystal X-ray diffraction: XD-3A (Japan); please refer to J. Org. Chem. 2008, 73, 8305-8316 8305 for the detection method.

### Example 17: Preparation and Characterization of β-cyclodextrin Inclusion compounds of 3-thiomorpholinyl-8-oxo-8H-acenaphtho[1,2-b] pyrrole-9-carbonitrile

Firstly, 1.85g of β-cyclodextrin (1.63 mMol) being secondary recrystallized and fully dried were added into 100mL water, then heated and stirred until dissolved completely. After dissolving with 35 mL acetone, 0.179g of the compound to be detected (0.54 mMol) was added dropwise into the aqueous solution of β-cyclodextrin in lines. The mixture was heated and stirred for 3 days at a temperature of 60°C; as a result, some deposition separated out therefrom. Filtered, the filter cake was washed using a small quantity of distilled water. The free compound was washed out by a small quantity of acetone. A mauve solid was obtained after drying for 24 hours in vacuum at a temperature of 50°C.

The detection results using ultraviolet spectroscopy indicated that the ultraviolet absorption and solubility of the compound increased with the increase of the concentration of β-cyclodextrin. This showed that the corresponding inclusion compounds had been formed.

It can be seen from the results of the phase solubility diagram that the solubility of the compound increased by 1.5 times from 0.21 µM to 0.36 µM.

The detection results using fluorescence spectroscopy indicated that the value of fluorescence spectra increased with the increase of the concentration of β-cyclodextrin under the condition that the concentration of the compound to be detected was kept invariably. When the fluorescence emission wavelength did not change, but the intensity increased, because after the entrance of the compound into the cyclodextrin cavity, the environmental change in the cavity protected the compound molecules in the excited state from coming into contact with large volume molecules and the quenching agent. The change in the fluorescence spectra suggested that the compound and β-cyclodextrin had formed the corresponding inclusion compound.

The detection results using circular dichroism spectroscopy indicated that in the presence of β-cyclodextrin, the induced circular dichroism of the compound to be detected exhibited a strong and positive cotton effect at 260 nm ∼ 375 nm and a weak and positive cotton effect at 400 nm ∼ 500 nm. This suggested that the induced circular dichroism effect had been produced after the entrance of the compound into the chiral cavity of β-cyclodextrin, which indicated that the inclusion compound had been formed.

The detection results using infrared spectroscopy indicated that β-cyclodextrin showed strong absorption bands at 3410.18 and 1029.22 cm⁻¹ and showed a series of characteristic absorption bands in the fingerprint region at 579-911cm⁻¹. The compound showed two sharp characteristic absorption bands at 2218.55 cm⁻¹ and 1625.08 cm⁻¹. The intensity of the characteristic absorption peak at 2219.13 cm⁻¹ and 1625.48 cm⁻¹ fell and a slight displacement occurred in the infrared spectrum. Meanwhile, a new sharp peak appeared at 1706 cm⁻¹, which indicated that the inclusion compound had been formed.

The detection results using thermogravimetric analysis indicated that β-cyclodextrin exhibited inflection points at 298°C and began to degrade. However, different from β-cyclodextrin, the inclusion compound exhibited inflection points at 269°C and began to degrade, which indicated that the inclusion compound had been formed. The content of the cyclodextrin was 73.1 %, therefore the inclusion model was 1:1.

The SEM results showed that 3-thiomorpholinyl-8-oxo-8*H*-acenaphtho[1,2-b]pyrrole -9-carbonitrile had a needle-shaped appearance, while β-cyclodextrin had a bigger square-shaped appearance. The SEM diagram of the mixture of the compound and β-cyclodextrin had a mixed appearance of needle and square shapes. However, the β-cyclodextrin inclusion compound had a regular diamond-shaped appearance, which was obviously different from the three types of the above-mentioned one. From the obvious difference of appearance, it could be seen that the inclusion compound had been formed.

### Preparation and Characterization of Other inclusion Compounds:

In the same manner as Example 17, other compounds in the same series were included by different kinds of cyclodextrins. The products were also characterized by ultraviolet spectroscopy, spectrofluorometric method, circular dichroism spectroscopy, infrared spectroscopy, thermogravimetric analysis and SEM in order to prove the formation of the inclusion compounds. The change of the solubility of the compounds and their inclusion compounds before and after inclusion were detected for comparison through a phase solubility experiment. The detailed results were shown in Table 1.

It can be seen from Table 1 that the solubility of the acenaphtho heterocyclic compounds included by different kinds of cyclodextrins was greatly increased when compared with that of the compounds per se.

**Table 1**

| Acenaphtho heterocyclic compounds for inclusion | Cyclodextrins | Solubility of compounds (µM) | Solubility of inclusion compounds (µM) |
|---|---|---|---|
| 3-thiomorpholinyl-8-oxo-8*H*-acenaph tho[1,2-b]pyrrole-9-carbonitrile | β-cyclodextrin | 0.21 | 0.36 |
| 3-(4-aminophenylthio)-8-oxo-8*H*-ace naphtho[1,2-b]pyrrole-9-carbonitrile | β-cyclodextrin | 0.25 | 0.60 |
| 4-(thienyl-2-methoxyl)-8-oxo-8*H*-ace naphtho [1,2-b]pyrrole-9-carbonitrile | β-cyclodextrin | 0.28 | 0.52 |
| 4-(thienyl-2-methylamino)-8-oxo-8*H*-acenaphtho [ 1,2-b]pyrrole-9-carbonitri le | β-cyclodextrin | 0.39 | 0.81 |
| 3-(4-bromophenylthio)-8-oxo-8*H*-ace naphtho [ 1,2-b]pyrrole-9-carbonitrile | β-cyclodextrin | 0.18 | 0.62 |
| 8-oxo-8*H*-acenaphtho[1,2-b]pyrrole-9 -carbonitrile | β-cyclodextrin | 0.12 | 0.4 |
| 3,6-di(4-bromophenylthio)-8-oxo-8*H*-acenaphtho[1,2-b]pyrrole-9-carbonitri le | β-cyclodextrin | 0.16 | 0.63 |
| 3-thiomorpholinyl-8-oxo-8*H*-acenaph tho[ 1,2-b]pyrrole-9-carbonitrile | γ-cyclodextrin | 0.21 | 0.78 |
| 3-(4-aminophenylthio)-8-oxo-8*H*-ace naphtho [1,2-b]pyrrole-9-carbonitrile | γ-cyclodextrin | 0.25 | 0.70 |
| 4-(thienyl-2-methoxyl)-8-oxo-8*H*-ace naphtho[1,2-b]pyrrole-9-carbonitrile | γ-cyclodextrin | 0.28 | 0.62 |
| 4-( thienyl-2-methylamino)-8-oxo-8*H* -acenaphtho [1,2-b]pyrrole-9-carbonitr ile | γ-cyclodextrin | 0.39 | 0.92 |
| 3-(4-bromophenylthio)-8-oxo-8*H*-ace naphtho[1,2-b]pyrrole-9-carbonitrile | γ-cyclodextrin | 0.18 | 0.80 |
| 6-(4-aminophenylthio)-8-oxo-8*H*-ace naphtho [1,2-b]pyrrole-9-carbonitrile | γ-cyclodextrin | 0.28 | 0.70 |
| 3,6-di(4-aminophenylthio)-8-oxo-8*H*-acenaphtho[1,2-b]pyrrole-9-carbonitrile | γ-cyclodextrin | 0.31 | 0.95 |
| 3-(*p*-methylphenoxy)-8-oxo-8*H*-acena phtho[1,2-b]pyrrole-9-carbonitrile | 2-hydroxypropyl-β-cyclodextrin | 0.25 | 0.96 |
| 3-(4-aminophenylthio)-8-oxo-8*H*-ace naphtho[1,2-b]pyrrole-9-carbonitrile | 2-hydroxypropyl-β-cyclodextrin | 0.25 | 0.90 |
| 3-phenoxy-8-oxo-8*H*-acenaphtho[1,2b]pyrrole-9-carboxylate | 2-hydroxypropyl-β-cyclodextrin | 0.15 | 0.62 |
| 3-(4-methoxyphenoxy)-8-oxo-8*H*-ace naphtho[1,2-b]pyrrole-9-carbonitrile | 2-hydroxypropyl-β-cyclodextrin | 0.32 | 0.92 |
| 4-(4-isopropylphenoxy)-8-oxo-8*H*-ac enaphtho[1,2-b]pyrrole-9-carbonitrile | 2-hydroxypropyl-β-cyclodextrin | 0.12 | 0.60 |
| 3-(4-bromophenylthio)-8-oxo-8*H*-ace naphtho[1,2-b]pyrrole-9-carbonitrile | 2-hydroxypropyl-β-cyclodextrin | 0.18 | 0.91 |
| 6-(thienyl-2-methoxy)-8-oxo-8*H*-acen aphtho[1,2-b]pyrrole-9-carbonitrile | methyl-β-cyclodext rin | 0.35 | 0.52 |
| 6-(tetrahydro-2*H*-pyranyl-4-oxy)-8-ox o-8*H*-acenaphtho[1,2-b]pyrrole-9-car bonitrile | methyl-β-cyclodext rin | 0.25 | 0.58 |
| 6-(thienyl-2-methylamino)-8-oxo-8*H*-acenaphtho[1,2-b]pyrrole-9-carbonitri le | methyl-β-cyclodext rin | 0.37 | 0.78 |
| 3-(4-bromophenylthio)-8-oxo-8*H*-ace naphtho[1,2-b]pyrrole-9-carbonitrile | methyl-β-cyclodext rin | 0.18 | 0.35 |
| 3-(4-aminophenylthio)-8-oxo-8*H*-ace naphtho[1,2-b]pyrrole-9-carbonitrile | methyl-β-cyclodext rin | 0.25 | 0.61 |
| 6-(4-aminophenylthio)-8-oxo-8*H*-ace naphtho[1,2-b]pyrrole-9-carbonitrile | methyl-β-cyclodext rin | 0.28 | 0.85 |
| 3-(4-bromophenylthio)-8-oxo-8*H*-ace naphtho[1,2-b]pyrrole-9-carbonitrile | hydroxypropyl-γ-cy clodextrin | 0.18 | 0.72 |
| 3-phenylthio-8-oxo-8*H*-acenaphtho[1, 2-b]pyrrole-9-carbonitrile | hydroxypropyl-γ-cy clodextrin | 0.22 | 0.58 |
| 3-(4-bromophenylthio)-8-oxo-8*H*-ace naphtho[1,2-b]pyrrole-9-carbonitrile | hydroxypropyl-γ-cy clodextrin | 0.39 | 0.98 |
| 3-(4-bromophenylthio)-8-oxo-8*H*-acenaphtho[1,2-b]pyrrole-9-carbonitrile | hydroxypropyl-γ-cyclodextrin | 0.18 | 0.48 |
| 3-(4-aminophenylthio)-8-oxo-8*H*-ace naphtho[1,2-b]pyrrole-9-carbonitrile | hydroxypropyl-γ-cy clodextrin | 0.25 | 0.81 |
| 6-(4-aminophenylthio)-8-oxo-8*H*-ace naphtho[1,2-b]pyrrole-9-carbonitrile | hydroxypropyl-γ-cy clodextrin | 0.28 | 0.96 |

### Example 18: Preparation and Characterization of γ-cyclodextrin Complex of 3-thiomorpholinyl-8-oxo-8H-acenaphtho[1,2-b]pyrrole-9-carboxylic acid

3-thiomorpholinyl-8-oxo-8*H*-acenaphtho[1,2-b]pyrrole-9-carboxylic acid (0.263g, 0.75mmol) and N,N'- carbonyldiimidazole (0.179g) were dissolved into 3mL DMSO. After the mixture was stirred for 30 minutes at room temperature, γ-cyclodextrin (0.6485g, 0.5mmol) and 4mL triethanolamine were added thereinto. The reaction was lasted for 18 hours at room temperature. After the reaction was completed, about 200mL acetone was added into the reagent. Deposition separated out under decompression. The resulting deposition was purified using an ion exchange column, and the resulting product was washed with the mixed solvents of methanol and water, then 0.42g 3-thiomorpholinyl-8-oxo-8H-acenaphtho[1,2-b]pyrrole-9-carboxylic acid/γ-cyclodextrin complex was obtained after the solution was freeze-dried with a yield of 25%.

The detection results from H nuclear magnetic resonance and mass spectrum showed: 1H NMR (400M, D2O-d6) δ (ppm) 8.87 (d, J = 8.5 Hz, 1H) , 8.58-8.55 (m, 2H), 7.91 (t, J = 8.5 Hz, 1H), 7.39 (d, J = 8.5 Hz, 1H), 5.03(m , 8H), 3.83(m, 8H), 3.80(m, 8H ), 3.74(m, 8H), 3.72-3.70 (m, 3-N(CH₂*)₂(CH₂)₂S, 4H), 3.59(m, 8H), 3.52 (m, 8H), 2.98-2.96(m, 3-N(CH2)₂(CH₂)₂*S, 4H); (ESI) m/z (M+H)- (1629 m/z).

The results obtained by single-crystal X-ray diffraction showed that γ-cyclodextrin exhibited a series of sharp peaks at 12° and 15 ∼ 23°, while the compound 3-thiomorpholinyl-8-oxo-8*H*-acenaphtho[1,2-b]pyrrole-9-carboxylic acid only exhibited sharp peaks at 11° and 7°. However, the complex exhibited a new sharp peak at 6°, and the sharp peak at 11° disappeared, meanwhile, the complex exhibited a series of sharp peaks at 14 ∼ 18° and 20 ∼ 25°. The complex had new sharp peaks relative to the compound and cyclodextrin, which indicated that the complex had been formed.

After the compound 3-thiomorpholinyl-8-oxo-8*H*-acenaphtho[1,2-b] pyrrole-9-carboxylic acid was complexed by γ-cyclodextrin, the solubility in water obviously increased. The fitting equation of the phase solubility curve was Y = 0.68 + 0.14 × X. The solubility of the compound 3-thiomorpholinyl-8-oxo-8*H*-acenaphtho [1,2-b] pyrrole-9-carboxylic acid in water increased by 16.5 times from the original 0.68µM to 11.2µM.

### Preparation and Characterization of Other Complexes:

In the same manner as Example 18, other compounds in the same series were complexed by different kinds of cyclodextrins. The products also were characterized by ultraviolet spectroscopy, spectrofluorometric method, circular dichroism spectroscopy, infrared spectroscopy, thermogravimetric analysis and SEM in order to prove the formation of the complexes. The change of the solubility of the compounds and their complexes before and after complexing was detected for comparison through phase solubility experiment. The detailed results are shown in Table 2.

It can be seen from Table 2 that the solubility of the acenaphtho heterocyclic compounds complexed by different kinds of cyclodextrins was greatly increased when compared with that of the compounds per se.

**Table 2**

| Acenaphtho heterocyclic compounds for complexation | Cyclodextrins | Solubility of compounds (µM) | Solubility of complexes (µM) |
|---|---|---|---|
| 3-(*p*-methylphenoxy)-8-oxo-8*H*-acenaphtho[1,2-b]pyrrole-9-carb oxamide | γ-cyclodextrin | 0.58 | 4.32 |
| 3-(4-bromophenylthio)-8-oxo-8 *H*-acenaphtho[1,2-b]pyrrole-9-c arboxylic acid | γ-cyclodextrin | 0.55 | 8.63 |
| 3-thiomorpholinyl-8-oxo-8*H*-ace naphtho[1,2-b]pyrrole-9-carboxa mide | γ-cyclodextrin | 0.65 | 9.85 |
| 3-(*p*-methylphenoxy)-8-oxo-8*H*acenaphtho[1,2-b]pyrrole-9-carb oxamide | 2-hydroxypropyl-β-cy clodextrin | 0.58 | 4.90 |
| 3-(4-bromophenylthio)-8-oxo-8 *H*-acenaphtho[1,2-b]pyrrole-9-c arboxylic acid | methyl-β-cyclodextrin | 0.55 | 4.80 |
| 3-thiomorpholinyl-8-oxo-8*H*-ace naphtho[1,2-b]pyrrole-9-carboxa mide | hydroxypropyl-γ-cycl odextrin | 0.65 | 10.20 |

### Part III: Detection of the physicochemical properties of acenaphtho heterocyclic compounds, cyclodextrin inclusion compounds and complexes thereof

### Example 19: Detection of BH3 analogous degree of the compounds by fluorescence polarization assay

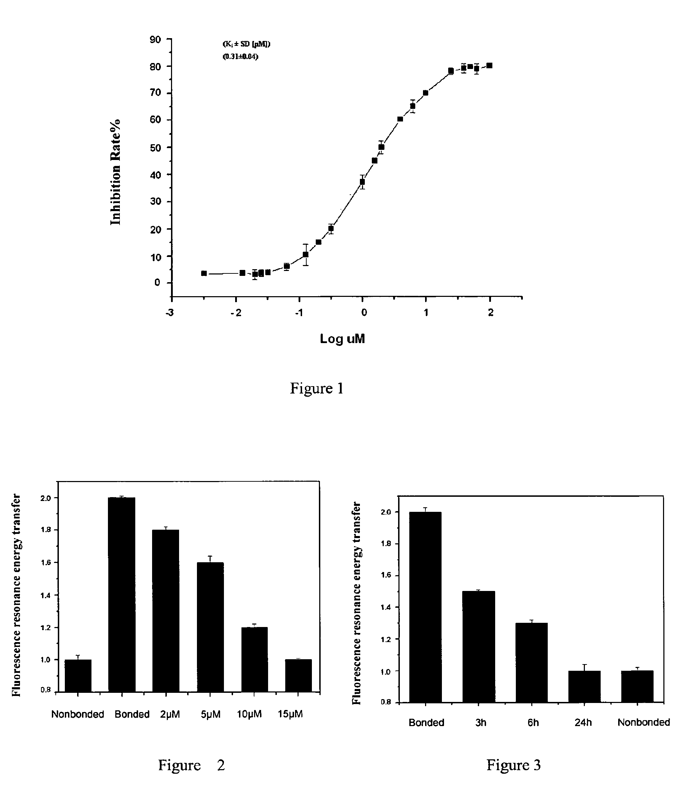

A Bid BH3 peptide (amino acids: 79-99: QEDIIRNIARHLAQVGDSMDR) having 21 amino acids was synthesized and marked with 6-carboxyfluorescein N-succinimidyl ester (FAM) as a fluorescent tag (FAM-Bid) at the N-terminal. The reaction system used in the competitive binding experiment was GST-Bcl-2 protein (40nM) or Mcl-1 protein, which was dissolved in the reaction buffer (100mM K3PO4, pH 7.5; 100µl g/ml bovine γ albumin ; 0.02% sodium azide) together with FAM-Bid polypeptide (5nM). In a 96-well plate, 1 00µL of the reaction system was added into each well. Then 1µL of various concentrations of 3-thiomorpholinyl-8-oxo-8*H*-acenaphtho[1,2-b]pyrrole-9-carbonitrile mother solution to be detected dissolved in DMSO was added thereinto until the final concentration met the experimental design requirements. Meanwhile, two control groups were established, one with the reaction system only containing Bcl-2 or Mcl-1 and FAM-Bid (equivalent to 0% inhibition rate), the other with the reaction system only containing FAM-Bid peptide. After 4 hours of incubation, the 96-well plate was detected by using an enzyme-labelled meter. The fluorescent polarization value (mP) was tested at 485nm emission wavelength excited and generated by 530nm wavelength. Ki value was deduced according to calculation formula. The experimental results are shown in Figure 1. The competitive binding constant between the compound and Bcl-2 is 310nM.

The BH3 analogous degrees of another 9 compounds were detected by using the experimental method as described above. The binding constant between them and Bcl-2 and Mcl-1 proteins were also on nM grade. The detailed results are shown in Table 3.

**Table 3**

| Compounds | Binding constant (nM) |
|---|---|
| 8-oxo-8*H*-acenaphtho[1,2-b]pyrrole-9- carbonitrile | 0.3 |
| 3-(4-bromophenylthio)-8-oxo-8*H*-acenaphtho[1,2-b]pyrrole-9 - carbonitrile | 17.0 |
| 6-(4-aminophenylthio)-8-oxo-8*H*-acenaphtho[1,2-b]pyrrole-9-carbonitrile | 20.0 |
| 3,6-di(4-bromophenylthio)-8-oxo-8*H*-acenaphtho[1,2-b]pyrrol e-9- carbonitrile | 5.0 |
| 3,6-di(4-aminophenylthio)-8-oxo-8*H-*acenaphtho[1,2-b]pyrrol e-9-carbonitrile | 7.0 |
| 3-(tetrahydro-2*H*-pyranyl-4-oxy)-8-oxo-8*H*-acenaphtho[1,2-b ]pyrrole-9- carbonitrile | 600.0 |
| 4-phenoxy-8-oxo-8H-acenaphtho[1,2-b]pyrrole-9- carbonitrile | 890.0 |
| 6-(thienyl-2-methoxy)-8-oxo-8*H*-acenaphtho[1,2-b]pyrrole-9-carbonitrile | 560.0 |
| 3-phenoxy-8-oxo-8*H*-acenaphtho[1,2-b]pyrrole-9-carboxylate | 790.0 |

### Example 20: Detection of the BH3 analogous degree of the compounds by intracellular fluorescence polarization energy transfer (FRET)

2µg of Bcl-2-CFP and Bax-YFP plasmids were transfected separately or simultaneously into Hela cells by using the calcium phosphate coprecipitation method, 24 hours later, the cells were inoculated in a 6-well plate (2×10⁵cells/well), and the compound 3-thiomorpholinyl-8-oxo-8*H*-acenaphtho[1,2-b]pyrrole-9-carbonitrile to be detected dissolved in DMSO was added thereinto until the final concentration (2, 5, 10 and 15µM) was achieved. 24 hours later (please refer to Figure 2), the cells were washed with PBS three times. The fluorescence value was detected by GENIOS fluorescence enzyme-labelled meter (TECAN, Swiss). In a time-dependent experiment, the transfected cells were inoculated in a 6-well plate, after that, 40µM of the compound were added thereinto. 3, 6 and 24 hours later (Figure 3), the fluorescence intensities were detected by a plate reader. As for the cell group in which only Bcl-2-CFP plasmid was transfected, the values of 475nm emission wave length and 433 nm excitation wave length were recorded. As for the cell group in which only Bax-YFP plasmid was transfected, the values at 527nm emission wave length and 505nm excitation wave length were recorded. As for the cell group in which Bcl-2-CFP and Bax-YFP plasmids were co-transfected, the values at 527nm and 475 emission wave length and 433nm excitation wave length were recorded. The ratio of fluorescence intensity at 527nm and 475nm emission wave lengths was FRET. The FRET for the control group in which the plasmid was solely transfected was set at 1.0. This means that the fluorescence polarization energy transfer did not occur for two proteins. In the cotransfected cells, the FRET increased up to 2.0 due to the interaction of Bcl-2 protein and Bax protein, and that the interference to the interaction between the two proteins increased and FRET decreased with an increase in drug concentration and time. The cellular vitality was detected by applying the MTT method. The experimental results are shown in Figures 2 and 3. When the concentration of the compound reached 2µM, the interaction between Bcl-2 and Bax can be interfered with after 3 hours, and the results showed a concentration-time dependent trend.

Another 7 compounds were detected by following the same experimental method as described above, it has been experimentally proven that all the compounds had the function of simulating BH3-only protein in cells and can obviously interfere with the interaction between Bcl-2 and Bax under different concentration and time conditions. The detailed results are shown in Table 4,wherein the concentration and time indicate that the detected compound interfered with the interaction between Bcl-2 and Bax at the given concentration for the given time period.

**Table 4**

| Compounds | Concentration (µM) | Time (hour) |
|---|---|---|
| 8-oxo-8*H*-acenaphtho[1,2-b]pyrrole-9-carbonitrile | 0.1 | 2 |
| 3-(4-bromophenylthio)-8-oxo-8*H*-acenaphtho[1,2-b]pyrr ole-9- carbonitrile | 0.5 | 2 |
| 6-(4-aminophenylthio)-8-oxo-8*H*-acenaphtho[1,2-b]pyrr ole-9-carbonitrile | 0.2 | 2 |
| 3,6-di(4-bromophenylthio)-8-oxo-8*H*-acenaphtho[1,2-b] pyrrole-9- carbonitrile | 1.0 | 2 |
| 3,6-di(4-aminophenylthio)-8-oxo-8*H*-acenaphtho[1,2-b] pyrrole-9-carbonitrile | 1.0 | 2 |
| 3-(3-fluorophenylformyl)-8-oxo-8*H*-acenaphtho [1,2-b]p yrrole-9- carbonitrile | 10.0 | 6 |
| 4-phenoxy-8-oxo-8*H*-acenaphtho[1,2-b]pyrrole-9-carbo nitrile | 10.0 | 6 |
| 6-(thienyl-2-methoxy)-8-oxo-8*H*-acenaphtho[1,2-b]pyrr ole-9- carbonitrile | 10.0 | 6 |
| 3-phenoxy-8-oxo-8*H*-acenaphtho[1,2-b]pyrrole-9-carbo xylate | 10.0 | 6 |

### Example 21: Detection of the BH3 analogous degree of the compounds by co-localization between Bax protein and chondriosome

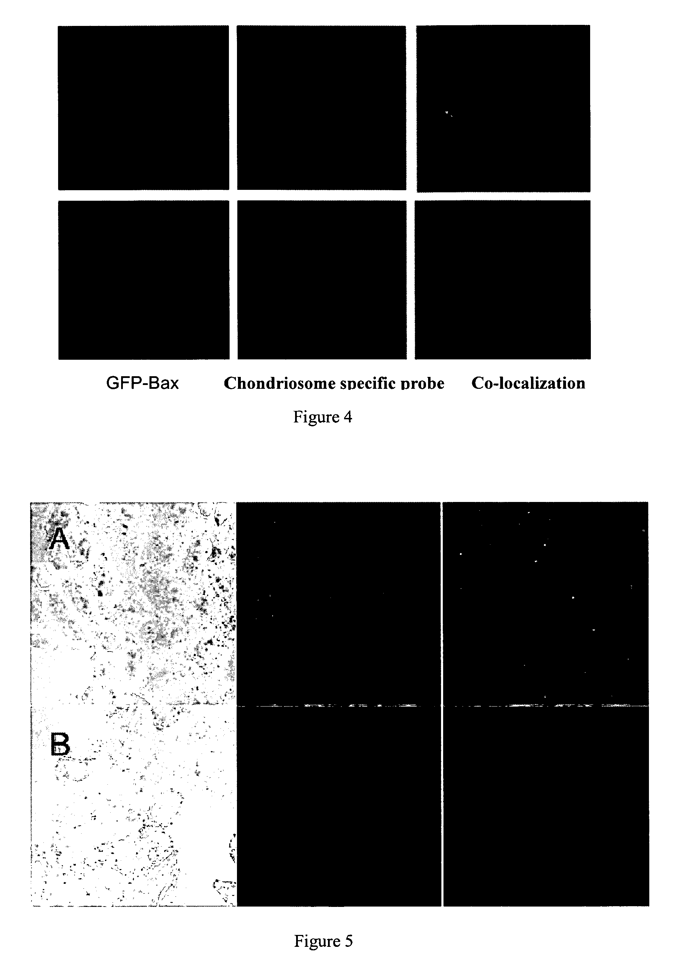

5µg of Bax-YFP plasmid trasfected into MCF-7 cells by using the calcium phosphate coprecipitation method; 24 hours later, the cells were inoculated in a 6-well plate (0.2×10⁶ cells/well), and 10µM of the compound 3-thiomorpholinyl-8-oxo-8 *H*-acenaphtho[1,2-b]pyrrole-9-carbonitrile to be detected were added thereinto. 6 hours later, the cells were washed with PBS and hatched in absence of light with 50nM Mito Tracker Red CMXRos (chondriosome specific probes; red) for 10 minutes. Then the cells were washed with PBS three times, and the fluorescent image was scanned with Radiance2000 laser confocal microscopy (Bio-Rad, USA). Meanwhile, dual channel scanning was carried out, one channel was used to scan the green fluorescence of Bax-YFP, and the other channel was used to scan the red fluorescence of the CMXRos probe for indicating the chondriosome. The co-localization circumstance was displayed by superimposing the two channel images. When the Bax protein was localized on the chondriosome, the green and red fluorescence was superimposed into orange, as shown in Figure 4. Figure 5, in comparison, shows that the BAX could not be driven to shift towards the chondriosome, i.e., the co-localization failed.

Another 8 compounds were detected by the same experimental method as described above. The results showed that all the compounds had the function of driving the BAX to shift towards the chondriosome, which indicates that they all had the function of simulating the BH3-only protein in cells. The detailed results are shown in Table 5wherein the concentration and time indicate that the detected compound simulated the BH3-only protein and drove the BAX to shift towards the chondriosome at the given concentration for the given time period.

**Table 5**

| Compounds | Concentration (µM) | Time (hour) |
|---|---|---|
| 8-oxo-8*H*-acenaphtho[1,2-b] pyrrole-9-carbonitrile | 1.0 | 3 |
| 3-(4-bromophenylthio)-8-oxo-8*H*-acenaphtho[1,2-b]py rrole-9- carbonitrile | 1.0 | 6 |
| 6-(4-aminophenylthio)-8-oxo-8*H*-acenaphtho[1,2-b]pyr role-9-carbonitrile | 5.0 | 6 |
| 3,6-di(4-bromophenylthio)-8-oxo-8*H*-acenaphtho[1,2-b ]pyrrole-9- carbonitrile | 5.0 | 6 |
| 3,6-di(4-aminophenylthio)-8-oxo-8*H*-acenaphtho[1,2-b ]pyrrole-9-carbonitrile | 5.0 | 6 |
| 4-phenoxy-8-oxo-8*H*-acenaphtho[1,2-b]pyrrole-9-carbonitrile | 10.0 | 6 |
| 6-(thienyl-2-methoxy)-8-oxo-8*H*-acenaphtho[1,2-b]pyr role-9- carbonitrile | 10.0 | 6 |
| 3-phenoxy-8-oxo-8*H*-acenaphtho[1,2-b]pyrrole-9-carb oxylic acid | 10.0 | 6 |

### Example 22: Experimental testing for the property of the BH3 analogues by the cytotoxicity of the compounds depending on BAX/BAK

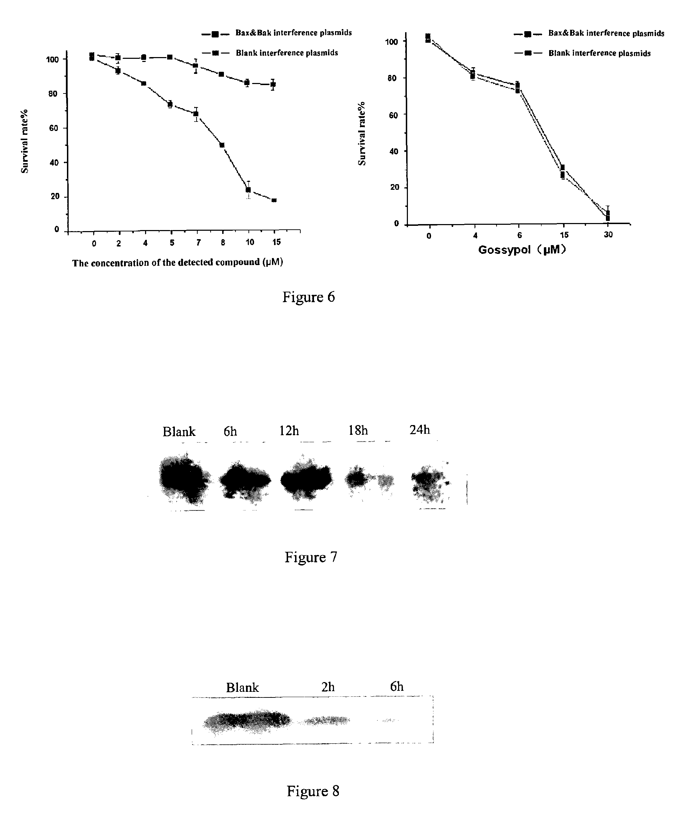

3µg of BAX/BAK interfering plasmid was trasfected into MCF-7 cells by using the calcium phosphate coprecipitation method; 24 hours later, the cells were collected. The expressions after the BAX and BAK proteins interfered with RNA was detected by Western, and the cell groups without plasmid transfection were treated similarly and were set as the control group. The transfected cells were inoculated in a 96-well plate (1× 10⁵ cells/well), the control experiment of the cell group without plasmid transfection was carried out in parallel. The compound 3-thiomorpholinyl-8-oxo-8 *H*-acenaphtho[1,2-b]pyrrole-9-carbonitrile to be detected was added thereinto according to the concentration gradient designed before the experiment. 48 hours later, the cellular vitality was detected by MTT. The experimental results are shown in Figure 6, Gossypol, as a nonspecific BH3 analogue, was treated in parallel. The results showed that the cytotoxicity of 3-thiomorpholinyl-8-oxo-8*H*-acenaphtho[1,2-b]pyrrole-9-carbonitrile was absolutely dependent on BAX/BAK.

Another 8 compounds (referred to as compounds ① ∼ ⑧) were also detected by following the same experimental method as described above; the results showed that the detected compounds also had the characteristics of absolute dependence on BAX/BAK. These compounds were as follows:
① 8-oxo-8*H*-acenaphtho[1,2-b]pyrrole-9- carbonitrile;
② 3-(4- bromophenylthio)-8-oxo-8*H*-acenaphtho[1,2-b]pyrrole-9- carbonitrile;
③ 6-(4-aminophenylthio)-8-oxo-8*H*-acenaphtho[1,2-b]pyrrole-9-carbonitrile;
④3,6-di(4-bromophenylthio)-8-oxo-8*H*-acenaphtho[1,2-b]pymole-9-carbonitrile;
⑤3,6-di(4-aminophenylthio)-8-oxo-8*H*-acenaphtho[1,2-b]pyrrole-9-carbonitrile;
⑥3-(3-fluorophenylformyl)-8-oxo-8*H*-acenaphtho[1,2-b]pyrrole-9- carbonitrile;
⑦ 6-(thienyl-2-methoxy)- 8-oxo-8*H*-acenaphtho[1,2-b]pyrrole-9- carbonitrile;
⑧ 3-phenoxy-8-oxo-8*H*-acenaphtho[1,2-b]pyrrole-9- carboxylic acid.

### Example 23: Detection of the inhibition of the compounds against Mcl-1 and Bcl-2 by Western blotting

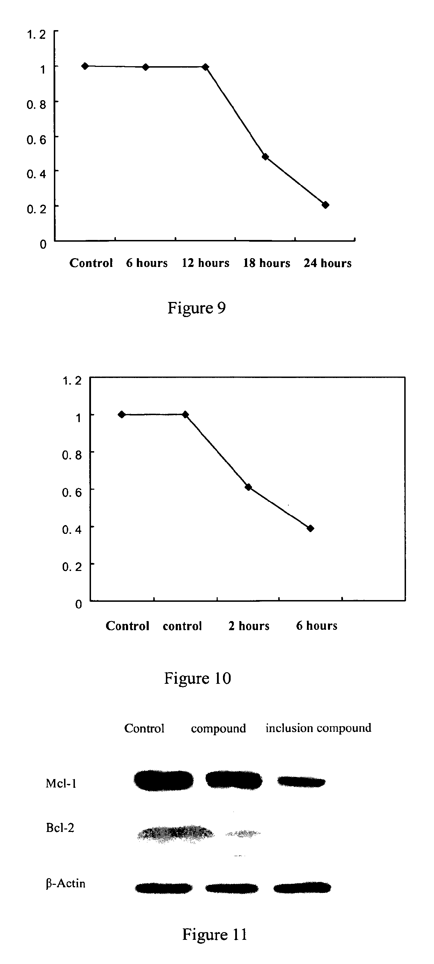

The cell sample was collected and cracked with 1×10⁶/50µl cell lysis solution (62.5mM Tris-HCL pH 6.8; 2%SDS; 10% glycerol; 50mM DTT; 0.01% bromphenol blue) at low temperature, then the solution was centrifuged and the protein supernatant was collected. The sample was boiled at 100°C for 5 minutes and then was separated by electrophoresis on 12% SDS-PAGE and transferred. The protein of interest was detected by the corresponding antibody. The expression of the interest protein in the cells was detected by horseradish peroxidase-labeled secondary antibodies in combination with the ECL coloration method. The inhibition of the compound 3-thiomorpholinyl-8-oxo-8*H*-acenaphtho[1,2-b]pyrrole-9-carbonitrile to be detected against Mcl-1 and Bcl-2 is separately shown in Figure 7 and Figure 8. It can be seen from the figures that the Bcl-2 and Mcl-1 protein bands gradually become lighter as time - for the compound to be detected acting on the tumor cells - progresses. This means that the compound had the function of inhibiting against these two proteins. The concentration of the protein bands in the Western images were carried out using semiquantitative analysis and normalization treatment with KODAK Gel Logic 1500 imaging system software. The concentration of the protein bands is shown in Figure 9 and Figure 10.

The following 8 compounds were also detected by using the same method as described above, it can be seen that they all had the inhibition against Bcl-2 and Mcl-1 proteins. These compounds comprise:
① 8-oxo-8*H*-acenaphtho[1,2-b]pyrrole-9- carbonitrile;
② 3-(4- bromophenylthio)-8-oxo-8*H*-acenaphtho[1,2-b]pyrrole-9- carbonitrile;
③ 6-(4-aminophenylthio)-8-oxo-8*H*-acenaphtho[1,2-b]pyrrole-9-carbonitrile;
④ 3,6-di(4-bromophenylthio)-8-oxo-8*H*-acenaphtho[1,2-b]pyrrole-9-carbonitrile;
⑤ 3,6-di(4-aminophenylthio)-8-oxo-8*H*-acenaphtho[1,2-b]pyrrole-9-carbonitrile;
⑥ 3-phenoxy-8-oxo-8*H*-acenaphtho[1,2-b]pyrrole-9-N-tert-butylamide;
⑦ 6-(thienyl-2-methoxy)- 8-oxo-8*H*-acenaphtho[1,2-b]pyrrole-9- carbonitrile;
⑧ 4-(tetrahydro-2*H*-pyranyl-4-oxy)-8-oxo-8H-acenaphtho[1,2-b]pyrrole-9-carbo nitrile.

The downregualation results of Mcl-1 protein and Bcl-2 protein for these compounds from semiquantitative analysis are shown in Table 6 and Table 7 respectively:

**Table 6: The downregulation results of Mcl-1 protein for compounds ①-⑧from semiquantitative analysis**

| Compounds | ① | ② | ③ | ④ | ⑤ | ⑥ | ⑦ | ⑧ |
|---|---|---|---|---|---|---|---|---|
| Control | 1 | 1 | 1 | 1 | 1 | 1 | 1 | 1 |
| 6 hours | 0.99 | 0.99 | 0.99 | 0.99 | 0.99 | 0.99 | 0.99 | 0.99 |
| 12 hours | 0.99 | 0.99 | 0.99 | 0.99 | 0.99 | 0.99 | 0.99 | 0.99 |
| 18 hours | 0.48 | 0.64 | 0.55 | 0.70 | 0.68 | 0.78 | 0.54 | 0.61 |
| 24 hours | 0.21 | 0.31 | 0.27 | 0.57 | 0.33 | 0.51 | 0.33 | 0.29 |

**Table 7: The downregulation results of Bcl-2 protein for compounds ①-⑧ from semiquantitative analysis**

| Compounds | ① | ② | ③ | ④ | ⑤ | ⑥ | ⑦ | ⑧ |
|---|---|---|---|---|---|---|---|---|
| Control | 1 | 1 | 1 | 1 | 1 | 1 | 1 | 1 |
| 2 hours | 0.78 | 0.66 | 0.79 | 0.68 | 0.72 | 0.80 | 0.69 | 0.71 |
| 6 hours | 0.69 | 0.35 | 0.49 | 0.49 | 0.43 | 0.56 | 0.40 | 0.38 |

### Example 24: Comparison of the inhibition against Mcl-1 and Bcl-2 among acenaphtho heterocyclic compounds and cyclodextrin inclusion compounds and complexes thereof by Western blotting

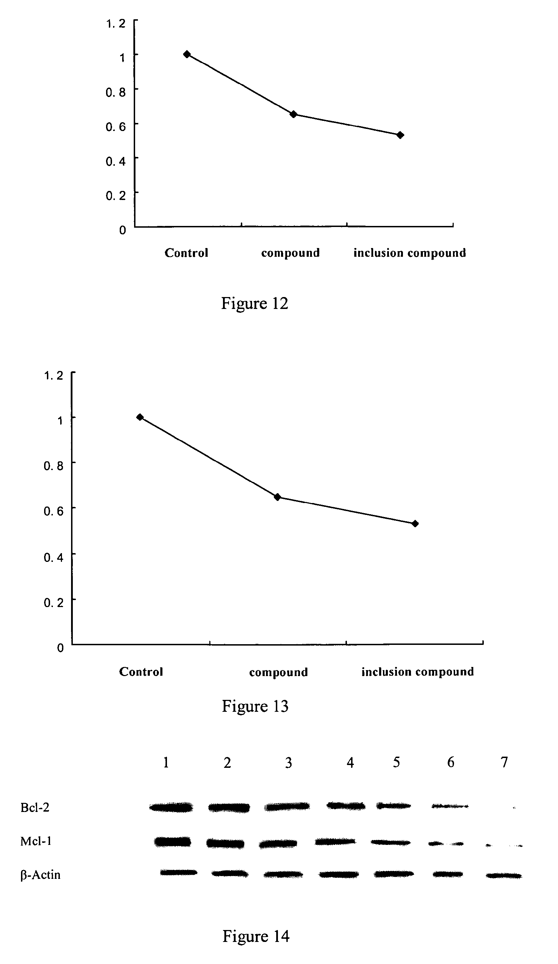

The cells were inoculated in a 6-well plate (2x 10⁵ cells/well). In the compound group, the compound 3-thiomorpholinyl-8-oxo-8*H*-acenaphtho[1,2-b]pyrrole-9-carbonitrile was dissolved into DMSO until the final concentration of 10µM was reached. In the inclusion group, γ-cyclodextrin inclusion, which was dissolved into water and was equivalent to 10µM of the compound, was added thereinto. 24 hours later, the cells were washed with PBS three times, then a cell sample was collected and cracked with 1 × 10⁶/50µl cell lysis solution (62.5mM Tris-HCL pH 6.8; 2%SDS; 10% glycerol; 50mM DTT; 0.01% bromphenol blue) at low temperature, then the solution was centrifuged and the protein supernatant was collected. The sample was boiled at 100°C for 5 minutes and then was separated by electrophoresis on 12% SDS-PAGE and transferred. The protein of interest was detected by the corresponding antibody. The expression of the protein of interest in the cells was detected by horseradish peroxidase-labeled secondary antibodies in combination with ECL coloration method. The detection results are shown in Figure 11. It can be seen from the figure that the inhibition of the γ-cyclodextrin inclusion compounds of 3-thiomorpholinyl-8-oxo-8*H*-acenaphtho[1,2-b]pyrrole-9-carbonitrile against Bcl-2 and Mcl-1 proteins was obviously higher than that of the compounds per se. That is to say, γ-cyclodextrin inclusion compounds obviously increased the inhibitory capability against Bcl-2 and Mcl-1 proteins. Concentration measurements of the protein bands in the Western figures were carried out by semiquantitative analysis and normalization treatment with KODAK Gel Logic 1500 imaging system software. The concentration of the protein bands is shown in Figure 12 and Figure 13.

In this example, 3-thiomorpholinyl-8-oxo-8*H*-acenaphtho[1,2-b]pyrrole-9-carbonitrile included by β-cyclodextrin, 2-hydroxypropyl-β-cyclodextrin, methyl-β-cyclodextrin and hydroxypropyl-γ-cyclodextrin and the following compounds were detected by using the same method as described above. The results showed that the included compounds had a higher inhibitory capability than that of the compounds themselves against Bcl-2 and Mcl-1 proteins in cells.
① 8-oxo-8*H*-acenaphtho[1,2-b]pyrrole-9- carbonitrile;
② 3-(4- bromophenylthio)-8-oxo-8*H*-acenaphtho[1,2-b]pyrrole-9- carbonitrile;
③ 6-(4-aminophenylthio)-8-oxo-8*H*-acenaphtho[1,2-b]pyrrole-9-carbonitrile;
④ 3,6-di(4-bromophenylthio)-8-oxo-8*H*-acenaphtho[1,2-b]pyrrole-9-carbonitrile;
⑤ 3,6-di(4-aminophenylthio)-8-oxo-8*H*-acenaphtho[1,2-b]pyrrole-9-carbonitrile;
⑥ 4-(tetrahydro-2H-pyranyl-4-oxy)-8-oxo-8*H*-acenaphtho[1,2-b]pyrrole-9-carbo nitrile.

The downregulation results of Mcl-1 protein and Bcl-2 protein for these compounds and their inclusion compounds from the semiquantitative analysis are shown respectively in Table 8 and Table 9:

**Table 8: The downregulation results of Mcl-1 protein for compounds ①—⑥ and their inclusion compound from the semiquantitative analysis**

| | ① | ② | ③ | ④ | ⑤ | ⑥ |
|---|---|---|---|---|---|---|
| Control | 1 | 1 | 1 | 1 | 1 | 1 |
| Compounds | 0.77 | 0.69 | 0.70 | 0.69 | 0.75 | 0.80 |
| Inclusion compounds | 0.63 | 0.35 | 0.49 | 0.57 | 0.60 | 0.66 |

**Table 9: The downregulation results of Bcl-2 protein for compounds ①—⑥ and their inclusion compounds from the semiquantitative analysis**

| | ① | ② | ③ | ④ | ⑤ | ⑥ |
|---|---|---|---|---|---|---|
| Control | 1 | 1 | 1 | 1 | 1 | 1 |
| Compounds | 0.69 | 0.67 | 0.74 | 0.69 | 0.75 | 0.77 |
| Inclusion compounds | 0.53 | 0.55 | 0.59 | 0.55 | 0.57 | 0.66 |

Other similar compounds, such as 3-(*p*-methylphenoxy)-8-oxo-8*H*-acenaphtho[1,2-b]pyrrole-9- carboxamide, 3-(4-bromophenylthio)-8-oxo-8*H*-acenaphtho[1,2-b]pyrrole-9- carboxylic acid, 3-thiomorpholinyl-8-oxo-8*H*-acenaphtho[1,2-b]pyrrole-9-carboxamide complexed by γ-cyclodextrin, 2-hydroxypropyl-β-cyclodextrin, methyl-β-cyclodextrin or hydroxypropyl-γ-cyclodextrin were detected in this example using the same method as described above. The experimental results showed that the cyclodextrin complexes of these compounds had a higher inhibitory capability than that of the compounds themselves against Bcl-2 and Mcl-1 proteins in cells.

### Example 25: Comparison of the inhibition against Mcl-1 and Bcl-2 between the compounds and inclusion compounds in tumor model in vivo by Western blotting

Kunming mice (in China) were randomly divided into groups, with 10 mice in each group. Cultivated hepatoma carcinoma cells H22 were inoculated subcutaneously in the oxter of the mice with 200µL/mouse. After bearing the cancer cells for five days, the subcutaneous tumor was formed. Then the compound 3-thiomorpholinyl-8-oxo-8*H*-acenaphtho[1,2-b]pyrrole-9-carbonitrile and γ-cyclodextrin inclusion compound thereof were given by peritoneal injection, and the compound to be detected included by γ-cyclodextrin was administered orally. The detection conditions comprise:
Blank control group: the mice were not treated in any manner after the bearing cancer;
Control group ①: the mice were given a peritoneal injection of DMSO solution every other day after bearing the cancer, 10 days in total;
Control group ②: the mice were given a peritoneal injection of cyclodextrin solution every other day after bearing the cancer, 10 days in total;
Experimental group ①: the mice were given a peritoneal injection of DMSO solution equivalent to 0.03 mg/kg BW compound every other day after bearing the cancer, 10 days in total;
Experimental group ②: the mice were given a peritoneal injection of DMSO solution equivalent to 0.3 mg/kg BW compound every other day after bearing the cancer, 10 days in total;
Experimental group ③: the mice were given a peritoneal injection of inclusion compound in an aqueous solution equivalent to 0.3 mg/kg BW compound every other day after bearing the cancer, 10 days in total;
Experimental group ④): of a water solution of inclusion compounds equivalent to 0.3 mg/kg BW compound was administered to the mice intragastrically every other day after bearing the cancer, 10 days in total;
During the experimental period, the length-diameter (a) and short diameter (b), which was perpendicular to the length-diameter, of the tumor were measured twice every week. The gross tumor volume was determined according to the formula: 1/2ab². The survival time of the animal was observed. The tumor inhibition rate was calculated by the tumor volume on the fortieth day. The results showed that:
Experimental group ② (injection group of the DMSO solution of compounds) : the tumor inhibition rate was 22.3%;
Experimental group ③ (injection group of the inclusion compounds): the tumor inhibition rate was 61.5%;
Experimental group④ (oral administration group of the inclusion compounds) : the tumor inhibition rate was 43.7%;

The average survival time of the animal in the control group was 28±2.1 days, the average life of the animal in the compound group was 33±3.1 days, the average survival time of the animal in the injection group of inclusion compounds was 48±5.1 days, and the average survival time of the animal in the oral administration group of inclusion compounds was 42±1.1 days. The result of the statistical treatment showed P<0.05.

After the mice died or were killed, the subcutaneous tumor was stripped. 1:3 volume of physiological saline was used to make tissue homogenate for the preparation of cell suspension. The expression of Bcl-2, Mcl-1 proteins in tumor cells was detected by using the same western detection method as described in example 24. The results are shown in Figure 14, wherein, the protein band in the electrophoresis path 6 was lighter than that in the electrophoresis path 5. This indicates that the inhibitory capability of the inclusion compounds against Bcl-2, Mcl-1 in vivo was higher than that of the compounds themselves.

3-thiomorpholinyl-8-oxo-8*H*-acenaphtho[1,2-b]pyrrole-9-carbonitrile included by β-cyclodextrin, 2-hydroxypropyl-β-cyclodextrin, methyl-β-cyclodextrin and hydroxypropyl-γ-cyclodextrin and the following compounds possessed the same antitumor effects in vivo, comprising:
① under the same conditions as experimental group ②, the tumor inhibition rate of 8-oxo-8*H*-acenaphtho[1,2-b]pyrrole-9-carbonitrile was about 60%; under the same condition as the experimental group ③, the tumor inhibition rate of the compound 29-1 included by γ-cyclodextrin was about 80%;
② under the same condition as experimental group②, the tumor inhibition rate of 3-(4-bromophenylthio)-8-oxo-8*H*-acenaphtho[1,2-b]pyrrole-9- carbonitrile was about 40%; under the same conditions as the experimental group③, the tumor inhibition rate of the compound 29-2 included by 2-hydroxypropyl-β-cyclodextrin was about 60%;
③ under the same conditions as experimental group ②, the tumor inhibition rate of 6-(4-aminophenylthio)-8-oxo-8*H*-acenaphtho[1,2-b]pyrrole-9-carbonitrile was about 30%; under the same condition as experimental group ③, the tumor inhibition rate of the compound 29-3 included by γ-cyclodextrin was about 40%;
④ under the same conditions as experimental group ②, the tumor inhibition rate of 3,6-di(4- bromophenylthio)-8-oxo-8*H*-acenaphtho[1,2-b]pyrrole-9- carbonitrile was about 78%; under the same conditions as experimental group ③, the tumor inhibition rate of the compound 29-4 included by methyl-β-cyclodextrin was about 85%;
⑤ under the same conditions as experimental group②, the tumor inhibition rate of 3,6-di(4-aminophenylthio)-8-oxo-8*H*-acenaphtho[1,2-b]pyrrole-9-carbonitrile was about 50%; under the same conditions as experimental group③, the tumor inhibition rate of the compound 29-5 included by γ-cyclodextrin was about 60%;
⑥under the same conditions as experimental group②, the tumor inhibition rate of 4-(tetrahydro-2*H*-pyranyl-4-oxy)-8-oxo-8*H*-acenaphtho[1,2-b]pyrrole-9-carbonitrile was about 40%; under the same conditions as experimental group③, the tumor inhibition rate of the compound 29-6 included by β-cyclodextrin was about 55%;

The tumor inhibition rate of the other compounds was between 30% and 50%. The tumor inhibition rate of the cyclodextrin inclusion compounds was generally higher than that of the compounds themselves (P<0.05).

The following compounds complexed by γ-cyclodextrin, 2-hydroxypropyl-β-cyclodextrin, methyl-β-cyclodextrin, hydroxypropyl-γ-cyclodextrin also showed a stronger inhibitory capability against Bcl-2 and Mcl-1 and higher tumor inhibition rate than that of the compounds themselves.

Wherein:
The tumor inhibition rate of 3-(*p*-methylphenoxy)-8-oxo-8*H*-acenaphtho[1,2-b] pyrrole-9-carboxamide was 30%, and when complexed by methyl-β-cyclodextrin, the tumor inhibition rate reached about 38%;
The tumor inhibition rate of 3-(4-bromophenylthio)-8-oxo-8*H*-acenaphtho[1,2-b] pyrrole-9-carboxylic acid was 45%, and when complexed by hydroxypropyl-γ-cyclodextrin, the tumor inhibition rate reached 55%;
The tumor inhibition rate of 3-thiomorpholinyl-8-oxo-8*H*-acenaphtho[1,2-b] pyrrole-9-carboxamide was 45%, and when complexed by 2-hydroxypropyl-β-cyclodextrin, the tumor inhibition rate reached 60%.

## Claims

1. An acenaphtho heterocyclic compound having the following structural formula: 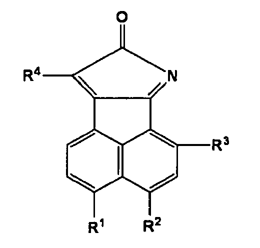 wherein:
(I) R¹=XR⁵, thiophene methoxyl, thiophene methylamino or thiomorpholinyl, R²=H, R³ =H, R⁴=CN COOH, COOR⁶ or CONHR⁷;
(II) R¹=H, R²=XR⁵, thiophene methoxyl, thiophene methylamino or thiomorpholinyl , R³=H, R⁴=CN, COOH, COOR⁶ or CONHR⁷;
(III) R¹=H, R²=H, R³=H, XR⁵, tetrahydropyran-4-oxy-, tetrahydrothiapyran-4-oxy-, thiophene methoxyl, thiophene methylamino or thiomorpholinyl , R⁴=CN;
(IV) R¹=XR⁵ R²=H, , R³=XR⁵ R⁴=CN;
wherein:
X=O, S, carbonyl, ester or amide;
R⁵= a: (CH₂)ₙAr *-(o,m,p)Y,* Y = CH₃, NO₂, Ph, F, Cl, Br, CF₃, OCH₃, SCH₃ or NH₂; n=0 ∼̃ 4;
b: tetrahydropyran or tetrahydrothiapyran;
R⁶ = CH₃ or C₂H₅;
R⁷ = CH₃, C₂H₅ or Ar.

2. The cyclodextrin inclusion compound of the acenaphtho heterocyclic compound according to claim 1, **characterized in that** the cyclodextrin inclusion compound is prepared by the following method:
① weigh an amount of cyclodextrin and add it into water, then heat while stirring until the saturated solution is formed, wherein the cyclodextrin is β-cyclodextrin, γ-cyclodextrin, 2-hydroxypropyl-β-cyclodextrin, methyl-β-cyclodextrin or hydroxypropyl-γ- cyclodextrin;
② weigh an amount of the acenaphtho heterocyclic compound for inclusion, wherein the mole ratio between the compound and cyclodextrin is 1:3-10;
③ dissolve the acenaphtho heterocyclic compound for inclusion into acetone with a concentration of 5-10 mg/mL, and the resulting solution is added dropwise into the water solution of cyclodextrin in lines, then heat and stir for 1-6 days under the temperature of 40-65°C until the deposition separates out therefrom;
④ filter the above-mentioned solution and wash the filter cake with a small amount of distilled water, then wash out the compounds in free state with a small amount of acetone. After drying under the temperature of 50-70°C and vacuum conditions for 24-48 hours, the cyclodextrin inclusion compound of the acenaphtho heterocyclic compound according to claim 1 is obtained.

3. The cyclodextrin complex of the acenaphtho heterocyclic compound according to claim 1, **characterized in that** the cyclodextrin complex is prepared by the following method:
① weigh dry cyclodextrin and the acenaphtho heterocyclic compound to be complexed, the mole ratio between the cyclodextrin and the acenaphtho heterocyclic compounds being 1:1.5-3; wherein the cyclodextrin is β-cyclodextrin, γ-cyclodextrin, 2-hydroxypropyl-β-cyclodextrin, methyl-β-cyclodextrin or hydroxypropyl-γ-cyclodextrin;
② mix the acenaphtho heterocyclic compound to be complexed with N,N'-carbonyldiimidazole with a mole ratio of 1:1-2, and then dissolve them into DMSO until the concentration of the acenaphtho heterocyclic compound to be complexed is 0.2-0.5mmol/mL in DMSO solution, then stir at room temperature for 30-60 minutes;
③ Add the cyclodextrin weighed in step ① and 0.1-0.3 mmol/mL of triethanolamine into the DMSO solution, and then let the reaction last for 18-24 hours at room temperature;
④ add 0.50-1.0 mg/mL of acetone into the reaction system of step 3, the deposition is separated out therefrom under decompression conditions;
⑤ filter, purify and the cyclodextrin complex of the acenaphtho heterocyclic compound of claim 1 is obtained.

4. Use of the acenaphtho heterocyclic compound according to claim 1 in the manufacture of BH3 analogue, Bcl-2 family protein inhibitors.

5. Use of the cyclodextrin inclusion compound of the acenaphtho heterocyclic compound according to claim 2 in the manufacture of BH3 analogue, Bcl-2 family protein inhibitors.

6. Use of the cyclodextrin complex of the acenaphtho heterocyclic compound according to claim 3 in manufacturing BH3 analogue, Bcl-2 family protein inhibitors.
